(19) Europäisches Patentamt
European Patent Office
Office européen des brevets

(11) **EP 2 497 830 A1**

(12) **EUROPEAN PATENT APPLICATION**
published in accordance with Art. 153(4) EPC

(43) Date of publication:
**12.09.2012 Bulletin 2012/37**

(51) Int Cl.:
*C12N 15/63* (2006.01)   *C12N 15/86* (2006.01)

(21) Application number: **10795406.7**

(22) Date of filing: **04.11.2010**

(86) International application number:
**PCT/ES2010/070715**

(87) International publication number:
**WO 2011/054994 (12.05.2011 Gazette 2011/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.11.2009 ES 200902122**

(71) Applicant: **Proyecto de Biomedicina Cima, S.L.**
**31008 Pamplona - Navarra (ES)**

(72) Inventors:
- **GONZÁLEZ ASEGUINOLAZA, Gloria**
  **E-31008 Pamplona - Navarra (ES)**
- **PRIETO VALTUEÑA, Jesús María**
  **E-31008 Pamplona - Navarra (ES)**
- **VANRELL MAJÓ, Lucía María**
  **E-31008 Pamplona - Navarra (ES)**

(74) Representative: **ABG Patentes, S.L.**
**Avenida de Burgos, 16D**
**Edificio Euromor**
**28036 Madrid (ES)**

(54) **REGULATED EXPRESSION SYSTEMS**

(57)    The application relates to gene constructs for inducible hepato- specific expression of polynucleotides of interest in response to an inducer agent, said constructs comprising (i) an inducible bi-directional operator -promoter with at least one responsive element to said inducer agent flanked by two hepato- specific promoters acting in divergent manner, (ii) a first nucleotide sequence encoding a transactivator which may be activated by said inducer agent operatively coupled to the first hepato- specific promoter and (iii) a second nucleotide sequence operatively coupled to the second hepato- specific promoter, wherein the promoters are induced as a consequence of the binding of the transactivator to the operator region of the operator -promoter in the presence of the inducer agent.

**EP 2 497 830 A1**

**Description**

**TECHNICAL FIELD OF THE INVENTION**

The invention belongs to the field of regulatable expression systems and, more specifically, of regulatable expression in spatial (in a given tissue) and temporal (in response to the addition of an inducer agent) form. The invention also relates to gene constructs and virions that allow for regulated hepato-specific expression, as well as the use thereof in the treatment of hepatic diseases.

**BACKGROUND OF THE INVENTION**

The functions of the liver include, amongst others, metabolism of carbohydrates and lipids, secretion of cytokines, elimination of insulin and other hormones, production of bile, etc. Moreover, factors that affect numerous genetic, cardiovascular, metabolic, haemorrhagic and cancerous diseases are produced in the liver. Liver cells have long half-lives and are directly connected to the blood stream, which facilitates the arrival of therapeutic agents thereto. For these reasons, the liver is considered to be a good candidate for gene therapy. However, in order to prevent the secondary effects associated with the expression of the target gene in non-hepatic tissues, it is convenient to have systems available which allow for the specific expression of a gene of interest in the liver.

To this end, constructs have been used wherein the gene of interest is under the control of a hepato-specific promoter such as the phosphoenolpyruvate carboxykinase promoter (PEPCK), gluconeogenesis enzymes (Yang, Y.W., J. et al., Gene Med., 5: 417-424 (2003)), α-1-anti-trypsin, albumin, FVII, organic anion transporter polypeptide (OATP-C), hepatitis B virus core protein (Kramer, M. G., et al., Mol. Ther., 7: 375-385, 2003), and thyroxine-binding globulin (Wang, L., et al., Proc. Natl. Acad. Sci, 96: 3906-3910 (1999). However, this type of systems has the disadvantage of leading to the constant expression of the gene of interest, which may result in toxicity and undesireable effects.

In order to prevent the effects resulting from the constant expression of the genes of interest, inducible systems have been developed wherein the expression takes place solely in the presence of a given inducer agent. Thus, an inducible expression system is known which may be controlled temporally and spatially, based on the use of a promoter that may be activated in the presence of a chimeric transcription factor the activity whereof is induced in the presence of mifepristone (RU-486) (see Wang et al., Nat. Biotechnol., 1997, 15: 239-43). This type of regulation may be applied to any cell type using a tissue-specific promoter. This system is specific, reversible and non-toxic. However, it has the disadvantage of leading to high expression levels under basal conditions, which makes it unacceptable if its *in vivo* application is desired.

Zabala et al. (Cancer Research 2004, 64: 2799-2804) have described plasmid vectors that include different embodiments of a tetracycline-inducible expression system (tet-on), all of which comprise a sequence that encodes a transgene (luciferase, or IL-12) which is transcribed from a transcription unit controlled by an operator-promoter sequence composed of 7 copies of the tetracycline Operator (tetO7) bound to the albumin promoter (Palb); this system also includes a sequence that encodes a reverse transactivator rtTA (rtTA2s-M2), under the control of hepato-specific promoter sequences, which are different depending on the embodiment, selected from EIIPα1AT (promoter of the human α-1-anti-trypsin gene, Pα1AT, fused with the region that enhances the hepatitis B virus core antigen, EII), Ea1bPα1AT (promoter of the human α-1-anti-trypsin gene, Pα1AT, fused with the region that enhances the albumin gene, Ealb) and Phpx (promoter of the human hemopexin gene). In this work, it was proven that the basal expression of the transgene was directly proportional to the strength of the hepato-specific promoter used to control the expression of rtTA; the capacity to induce the expression of the transgene was inversely proportional to the basal expression (maximum rate of induction when Phpx, the weakest promoter, was used); however, the maximum expression levels following the induction were directly proportional to the potency of the promoter, that is, the strongest promoter expressed the highest levels following the induction. On the other hand, when the rtTA transcription units and the transgene are placed in tandem, the expression of the transgene is greater than when they are placed in opposite directions. However, in this study, the use of a Palb promoter to direct the expression of the transgene was associated with a lower expression of the transgene, as compared to that obtained with a system that used the minimal cytomegalovirus promoter.

Kramer et al. (Molecular Therapy, 2003, 7: 375-385) compare the promoter activity of various promoter constructs, amongst them, the hepato-specific EIIPα1AT, EalbPa1AT and Phpx promoters. They prove that the Pa1AT promoter, by itself or bound to the Ealb or EII enhancers of the VHB virus, is the most potent to direct the stable expression of a gene in hepatic cells.

Chtarto et al. (Gene Therapy, 2003, 10: 84-94) describe an AAV vector for the inducible expression of a transgene (eGFP reporter gene, enhanced GFP) from a tetracycline-inducible bi-directional transgene expression system. Said system includes a sequence that contains the tetO7 operator region flanked on both sides by minimal cytomegalovirus promoter sequences (pCMVm) that direct, in opposite directions, the transcription of a transactivator (rtTA) which may be activated by tetracyclines and the transgene, such that, in the presence of doxycycline, the rtTA transactivator induces the transcription of the transgene and of itself. The system also includes bi-directional SV40 polyadenylation signals. This self-regulating system exhibits the capacity to induce the expression of a transgene in tumoural cell lines and *in vivo* expression in the brain. In a more recent report (Chtarto et al. Experimental Neurology 2007, 204: 387-399), this same group describes an improved version of the vector, which carries a mutated rtTA transactivator, and which makes it possible to express GDNF in the striated nucleus in biologically active concentrations that repress tyrosine-hydroxylase in rats treated with doxycycline, but not in non-induced controls. However, there are no data in regards to whether these vectors are suitable to obtain transgene expression in the liver with the requirements specified above.

However, the state of the art needs to develop alternative systems for the inducible hepato-specific expression of genes of interest that do not present the disadvantages of the systems described thus far.

## SUMMARY OF THE INVENTION

The authors of this invention have shown that inducible expression vectors based on the use of tetracycline-sensitive transactivators and minimal CMV promoter do not exhibit good behavior for controlled specific expression in the liver, but, on the contrary, heterologous gene expression systems that include human albumin promoters (pA1b) instead of minimal pCMV promoters make it possible not only to obtain a lower liver-specific basal expression, but, surprisingly, also make it possible, following the induction, to obtain expression levels that are greater than those obtained with stronger promoters of the minimal CMV type.

Thus, a first aspect of this invention relates to a gene construct that allows for the inducible hepato-specific expression of a polynucleotide of interest in response to an inducer agent, which comprises

(i) an inducible bi-directional operator-promoter that comprises at least one responsive element to said inducer agent flanked by a first hepato-specific promoter sequence and a second hepato-specific promoter sequence, wherein both hepato-specific promoter sequences act in a divergent manner,
(ii) a first nucleotide sequence that comprises a sequence that encodes a transactivator that may be activated by said inducer agent and a polyadenylation signal located at the 3' position with respect to the region that encodes the transactivator, wherein said sequence that encodes a transactivator is operatively coupled to the first hepato-specific promoter sequence and
(iii) a second nucleotide sequence that comprises a polynucleotide that is operatively coupled to the second hepato-specific promoter sequence and a polyadenylation signal located at the 3' position with respect to the polynucleotide of interest,

wherein the promoter activity of said first and second hepato-specific promoter sequences is induced as a consequence of the binding of the transactivator to the operator region of the operator-promoter in the presence of the inducer agent.

A second aspect of the invention relates to a vector, a viral genome or a virion that comprises the gene construct of the invention.

Another aspect of the invention relates to a virion obtainable by expressing a viral genome of the invention in a suitable packaging cell.

Another aspect of the invention relates to an *in vitro* method for the expression of a polynucleotide of interest in a cell of hepatic origin, which comprises the following steps:

(i) placing said cell in contact with a gene construct in accordance with the invention, a vector in accordance with the invention, a viral genome in accordance with the invention or a virion in accordance with the invention, under adequate conditions for the entry of said construct, said vector or said virion into the cell, and
(ii) placing the cell in contact with the inducer agent for the time necessary to produce the expression of the polynucleotide of interest.

Additional aspects of the invention relate to a pharmaceutical composition a gene construct in accordance with the invention, to a vector in accordance with the invention, to a viral genome in accordance with the invention or to a virion in accordance with the invention, as well as the use thereof as a drug or to be used in the treatment of a hepatic disease.

Another aspect of the invention relates to an inducible bi-directional operator-promoter suitable for the inducible hepato-specific expression of two polynucleotides of interest by an inducer agent, which comprises

(i) at least one responsive element to said inducer agent,
(ii) a first hepato-specific promoter sequence and
(iii) a second hepato-specific promoter sequence,

wherein the first and the second hepato-specific promoter sequences act in a divergent manner with respect to the responsive element to the inducer agent and wherein the promoter activity of the first and the second hepato-specific promoter sequences increases in the presence of said inducer agent and in the presence of a transactivator that binds to the responsive element.

An additional aspect of the invention relates to a gene construct suitable for the inducible hepato-specific expression of a polynucleotide of interest by an inducer agent, which comprises

(a) an inducible bi-directional operator-promoter that comprises

(i) at least one responsive element to said inducer agent,
(ii) a first hepato-specific promoter sequence and
(iii) a second hepato-specific promoter sequence,

(b) a nucleotide sequence that encodes a transactivator which may be activated by said inducer agent that is operatively coupled to the first hepato-specific promoter sequence and a polyadenylation signal located at the 3' position with respect to the region that encodes the transactivator,

wherein the first and the second hepato-specific promoter sequences act in a divergent manner with respect to the responsive element to the inducer agent and wherein the promoter activity of the first and the second hepato-specific promoter sequences increases in the presence of said inducer agent and in the presence of a transactivator that binds to the responsive element in the inducible bi-directional operator-promoter.

## DESCRIPTION OF THE FIGURES

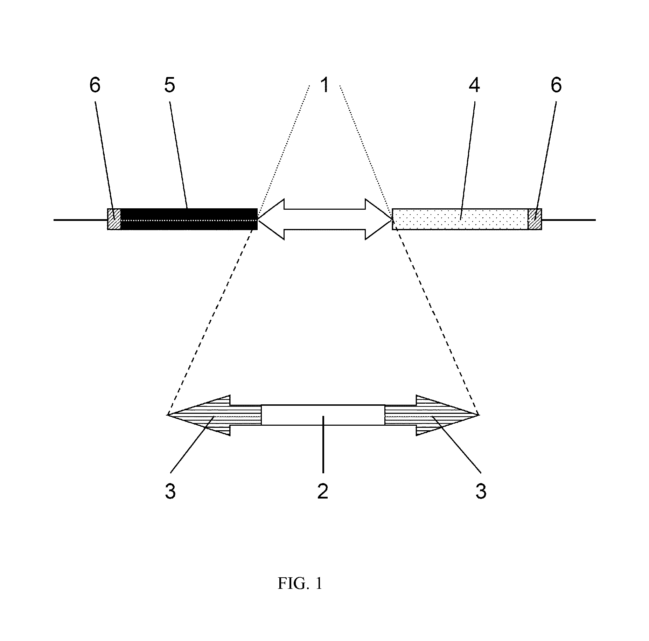

Figure 1. Diagram of the structure of the tetracycline-inducible transgene expression system of the invention. (1) bi-directional operator-promoter sequence; (2) bi-directional responsive element to the active form of the transactivator; (3) promoter sequences that comprise a hepato-specific promoter (preferably an albumin promoter (pA1b) or a minimal albumin promoter (pmA1b)); (4) sequence that encodes a reverse transactivator which may be activated by an inducer agent, preferably by tetracyclines (rtTA); (5) sequence that encodes a transgene of interest; (6) polyadenylation signals (polyA or pA). The bi-directional operator-promoter sequence controls the transcription of the sequences that encode the transactivator (preferably rtTA) (4) and the transgene of interest (5); in turn, its promoter activity is induced by the transactivator protein (preferably rtTA) (4) in the presence of the inducer agent (preferably tetracycline or an analogue of tetracycline such as doxycycline).

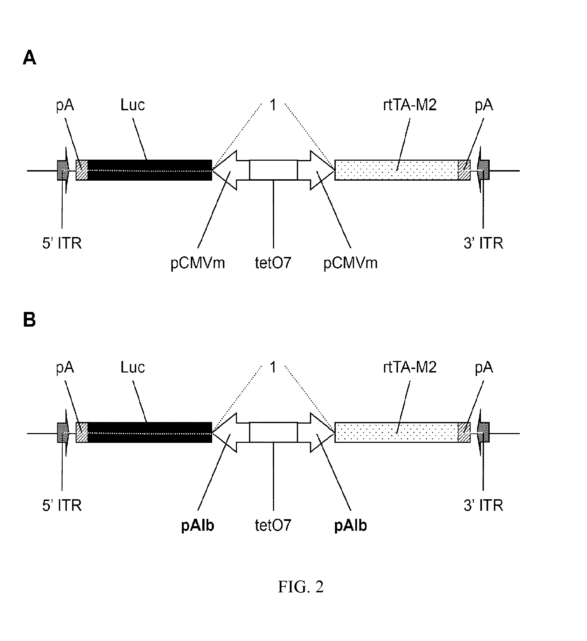

Figure 2. Structure of different recombinant adeno-associated viruses used in the examples, wherein a tetracycline-inducible transgene expression system has been incorporated.

A) rAAV-pTet_{bidi}-pCMV-luc: The genome of this adeno-associated virus has a tetracycline-inducible bi-directional expression system incorporated which includes an operator region with 7 copies of 42 bases of the tetracycline Operator (tetO7), flanked by 2 minimal cytomegalovirus promoters (pCMV); the operator-promoter controls the expression of 2 sequences, one placed on each side, which encode, respectively, a reverse transactivator rtTA-M2 and luciferase (luc) as the transgene of interest; the bi-directional SV40 polyadenylation signals have been incorporated as the polyadenylation signals (pA); the 5'-ITR (inverted terminal repeat) and the 3' ITR of the adeno-associated virus type 2 (AAV2) have been included flanking the expression cassette.

B) rAAV-pTet_{bidi}-pA1b-luc: The genome of this adeno-associated virus has a tetracycline-inducible bi-directional expression system incorporated which includes the same elements as the rAAV-pTet_{bidi}-pCMV-luc virus, with the sole difference that the minimal pCMV promoters have been replaced with the albumin gene promoter (pA1b) sequences.

C) rAAV-pTet_{bidi}-pA1b-mIL12: The genome of this adeno-associated virus has a tetracycline-inducible bi-directional expression system incorporated which includes the same elements as the rAAV-pTet_{bidi}-pA1b-luc virus, with the sole difference that the luciferase gene has been replaced with the mouse single-chain IL12 sequence (Lieschke, G.J., et al.m Nat Biotechnol, 1997. 15: 35-40).

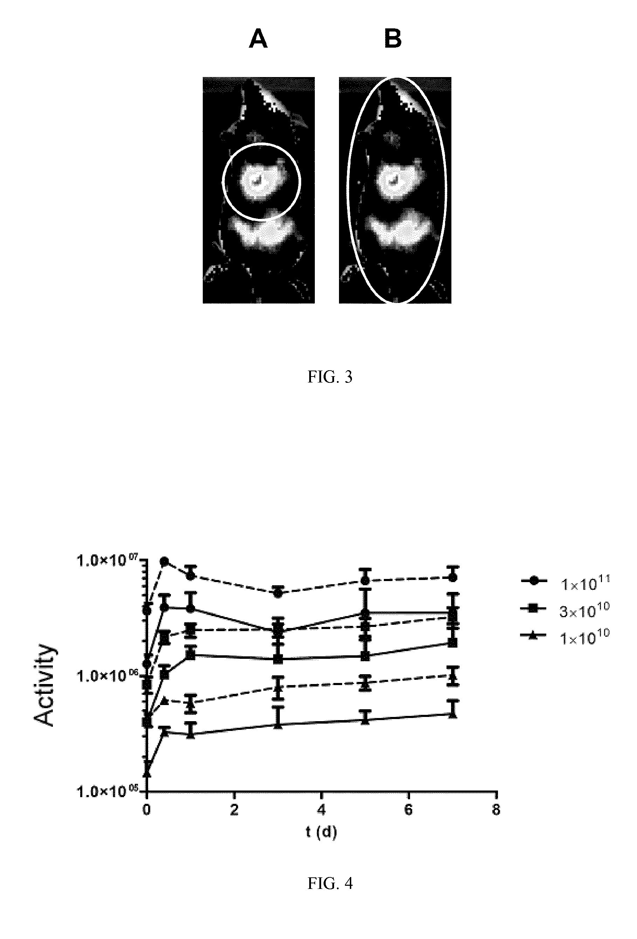

Figure 3. Bioluminescence images obtained by means of a CCD camera. They show the regions that are selected to measure luciferase activity levels. A) Upper abdominal area (includes the liver), and B) Levels of bioluminescence emitted by the entire animal.

Figure 4. Measurement of the luciferase activity (photons/s) in female BALB/c mice injected with virions or viral particles containing genomes of the recombinant rAAV-pTet$_{bidi}$-pCMV-luc virus (in doses of 1 x $10^{10}$, 3 x $10^{10}$ and 1 x $10^{11}$ viral genomes (vg) per mouse, depending on the groups). The virions injected were AAV2/8 virions, which contained genomes constructed on AAV2 virus ITRs, but packaged in AAV8 capsids (composed of capsid proteins corresponding to an AAV of serotype 8). In order to induce the expression of luciferase, 21 days after the administration of the viral vector, each animal was administered doxycycline (50 mg/kg of weight; i.p. route), and the induction was maintained after 24 hours by the administration of doxycycline (dox) for 7 days in the drinking water (2 mg/ml of doxycycline; 5% sucrose). The lines indicate the luciferase activity measured as a function of time, expressed in days t(d) from the first i.p. administration of doxycycline (day 0). The activity levels in the hepatic area are represented with solid lines; the activity levels in the entire animal are represented with broken lines.

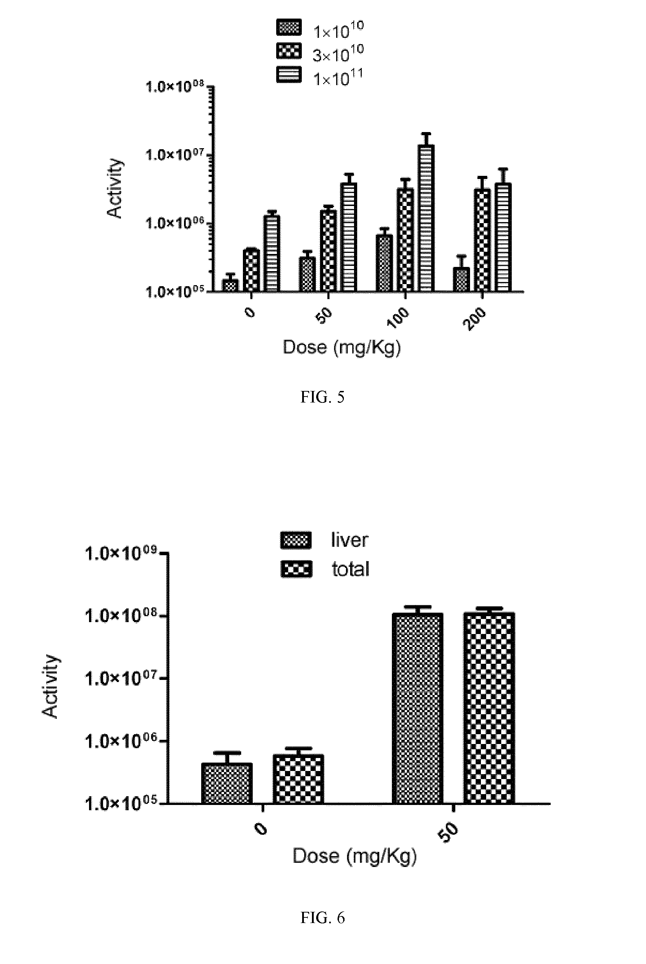

**Figure 5.** Measurement of the luciferase activity (photons/s) in mice injected with AAV2/8 virions that contained genomes of the rAAV-pTet$_{bidi}$-pCMV-luc virus (doses of 1 x $10^{10}$, 3 x $10^{10}$ and 1 x $10^{11}$ vg/mouse). The measurements were performed following repeated inductions with increasing doses of doxycycline (mg/kg; i.p. route), separated by a 15-day period. The luciferase activity was measured in the upper abdominal or hepatic area after 24 hours had elapsed since the i.p. administration of doxycycline.

**Figure 6**. Luciferase activity (photons/s) measured in female BALB/c mice injected, by intravenous route, with AAV2/8 virions that contained genomes of the recombinant rAAV-pTet$_{bidi}$-pA1b-luc virus (1 $\times$ $10^{11}$ vg/mouse). The activity was measured in the basal state prior to induction with doxycycline (Dose 0), and in the induced state 24 hours after the i.p. administration of 50 mg/kg of weight (Dose 50); these measurements were performed in both the upper abdominal area (Liver) and the entire animal (Total).

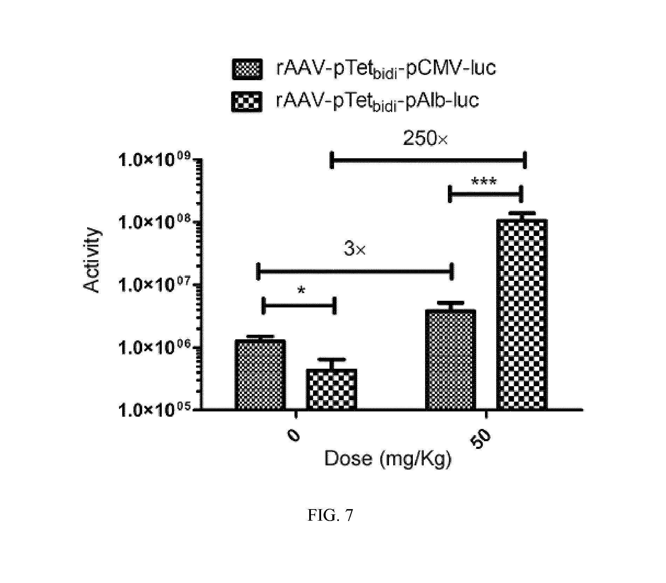

**Figure 7**. Comparison of the luciferase activities (photons/s) measured in the basal state (Dose 0) and in the induced state, 24 hours after the i.p. administration of 50 mg/kg of doxycycline (Dose 50) in female BALB/c mice injected with AAV2/8 virions that contained genomes of rAAV-pTet$_{bidi}$-pCMV-luc or rAAV-pTet$_{bidi}$-pA1b-luc (1 $\times$ $10^{11}$ vg/mouse; i.v. route). The measurements were performed in the upper abdominal area (hepatic).

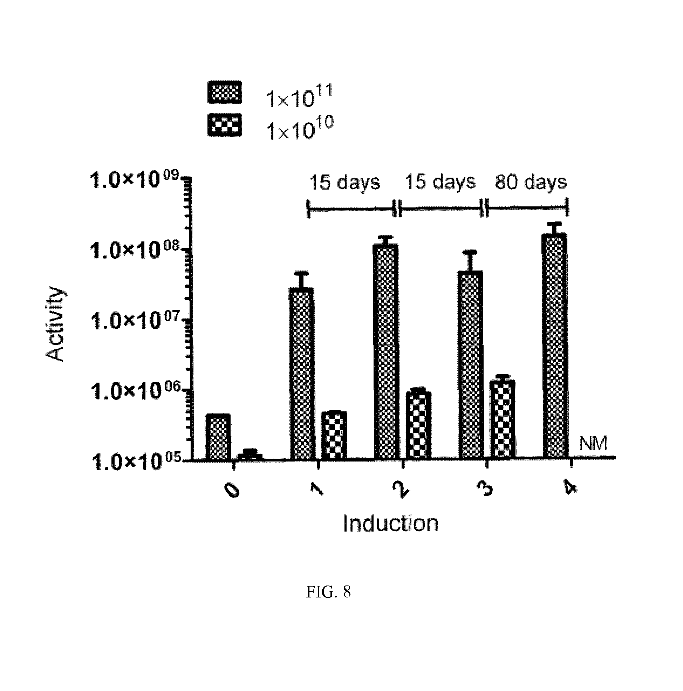

**Figure 8**. Luciferase activity (photons/s) in female BALB/c mice injected with AAV2/8 virions that incorporate genomes of the rAAV-pTet$_{bidi}$-pA1b-luc virus (at doses of 1 $\times$ $10^{11}$ and 1 $\times$ $10^{10}$ vg/mouse depending on the groups; i.v. route). The activity was measured in the basal state (Induction 0) and 24 hours after induction with 50 mg/kg of doxycycline in 4 repeated induction cycles (Inductions 1, 2, 3 and 4, respectively). Between induction 1 and induction 2, and between inductions 2 and 3, 15 days elapsed; between Induction 3 and Induction 4, 80 days elapsed.

**Figure 9.**

A) Luciferase activity (photons/s) of female and male C57BL/6 injected with AAV2/8 virions carrying genomes of rAAV-pTet$_{bidi}$-pA1b-luc (1 $\times$ $10^{11}$ vg/mouse; i.v. route), measured in the basal state and in the induced state, following induction with different doses of doxycycline. A different dose of doxycycline was administered to each group (N = 5) of mice (Doses in mg/kg of weight; i.p. route). The basal activity was measured at day 14 following the injection of the corresponding virus; the measurement in the induced state was performed 22 days after the injection of the virus and 24 hours after the administration of the doxycycline dose.
B) Logarithmic transformation [$\log_{10}$ (photons/s)] of the activity data shown in A).

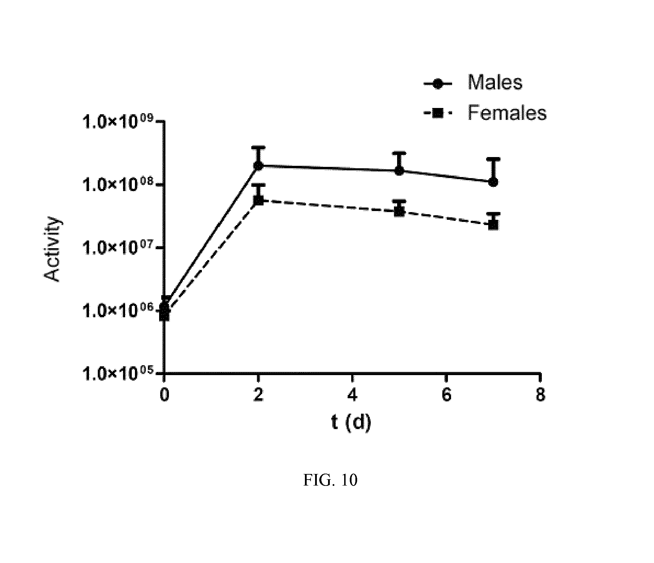

**Figure 10.** Luciferase activity (photons/s) measured in female and male C57BL/6 injected with AAV2/8 virions carrying rAAV-pTet$_{bidi}$-pA1b-luc (1 $\times$ $10^{11}$ vg/mouse; i.v. route), in the basal state (day 0) and at different days during the period of administration of doxycycline in the drinking water (2 mg/ml + 5% sucrose).

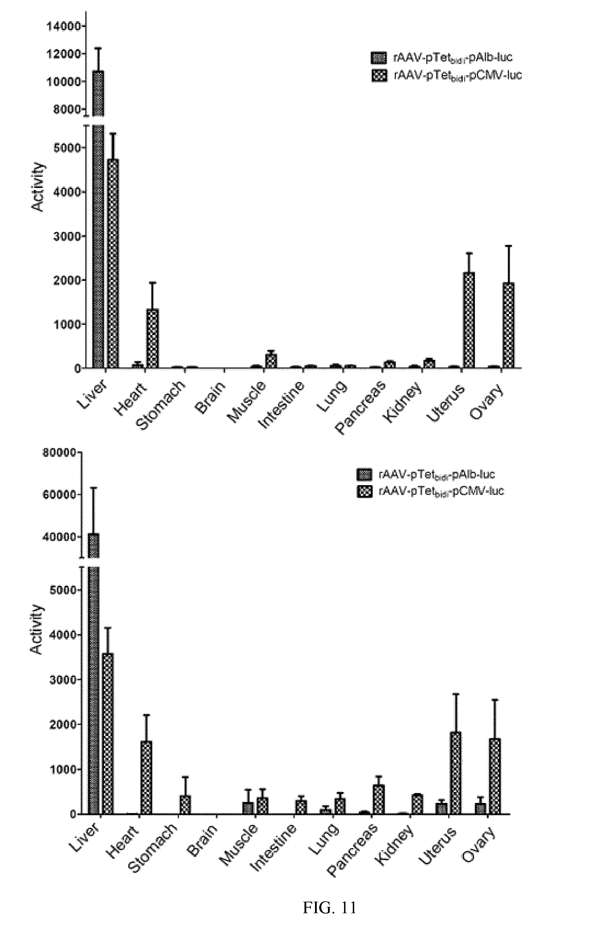

**Figure 11.** Biodistribution of the luciferase activity *ex vivo*. Female of the BALB/c (A) and C57BL/6 (B) strains (N = 4-8) were injected with AAV2/8 virions carrying rAAV-pTet$_{bidi}$-pCMV-luc or rAAV-pTet$_{bidi}$-pA1b-luc (a dose of 1 $\times$ $10^{11}$ vg/mouse, i.v. route). 21 days after the injection of the virus, the expression of luciferase was induced by the administration of doxycycline (50 mg/kg; i.p. route); 24 hours after the induction, the animals were sacrificed; the organs were extracted and the luciferase activity (RLU) was measured in each of them, normalising it with the amount of total protein (RLU/mg protein).

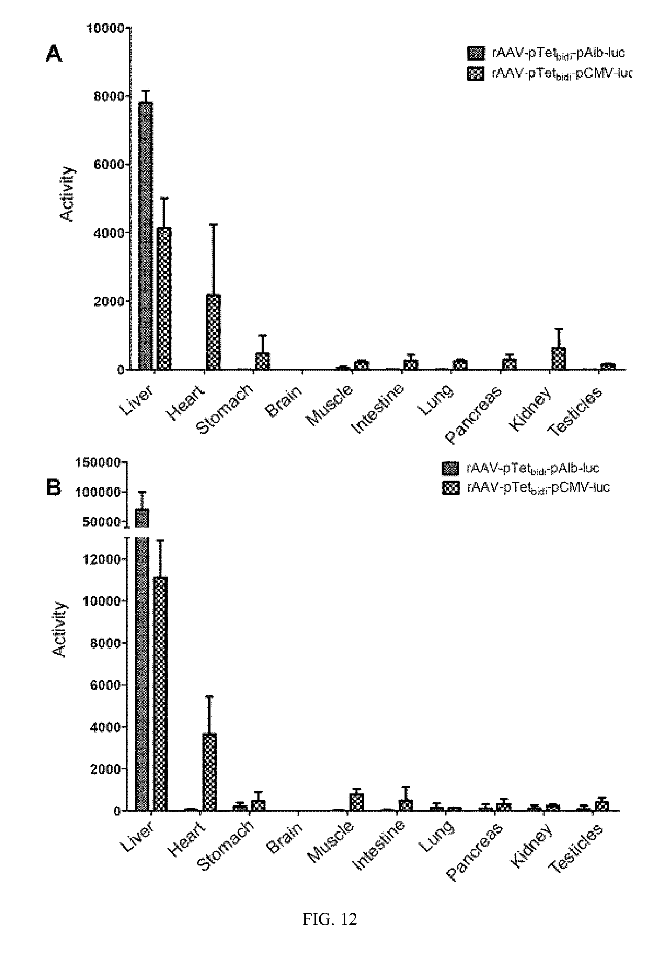

**Figure 12.** Biodistribution of the luciferase activity *ex vivo*. Male of the BALB/c (A) and C57BL/6 (B) strains (N = 4-8) were injected with AAV2/8 virions carrying rAAV-pTet$_{bidi}$-pCMV-luc or rAAV-pTet$_{bidi}$-pA1b-luc (a dose of 1 $\times$ $10^{11}$ vg/mouse by i.v. route). 21 days after the injection of the virus, the expression was induced by the administration of doxycycline (50 mg/kg; i.p. route); 24 hours after the induction, the animals were sacrificed; the organs were extracted and the luciferase activity (RLU) was measured in each of them, normalising it with the amount of total protein (RLU/mg protein).

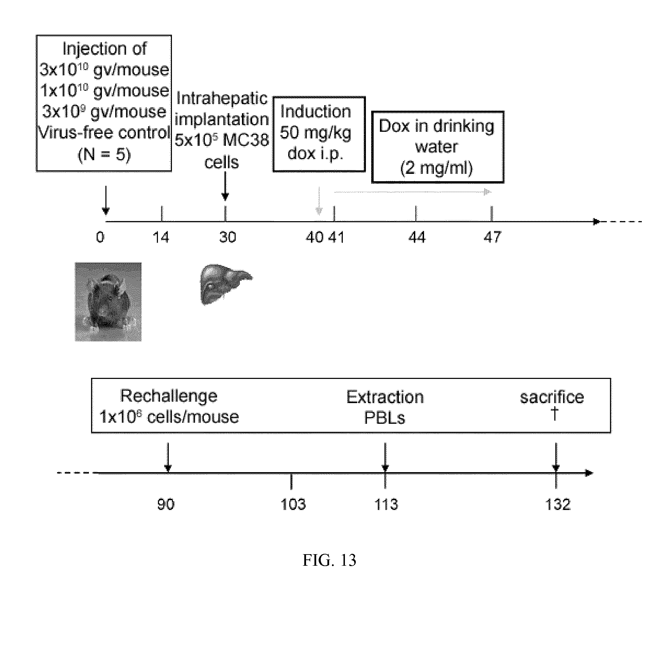

**Figure 13.** Diagram of the anti-tumour treatment protocol administered to female C57BL/6 mice following the implantation of cells from the MC38 syngeneic tumour line. At day 0, they were administered 3 different doses of AAV2/8 virions carrying rAAV-pTet$_{bidi}$-pA1b-mIL12 (3 $\times$ $10^{10}$, 1 $\times$ $10^{10}$ and 3 $\times$ $10^{9}$ vg/mouse), and a group of mice was left without a vector (N=5). After 30 days, 5 $\times$ $10^{5}$ MC38 cells were implanted intrahepatically. Ten days after the implantation (day 40 of the protocol), induction of the system began by means of the i.p. administration of doxycycline (50 mg/kg). On the following day, the induction was continued in the drinking water (2 mg/ml of dox + 5% sucrose), and it was maintained for the following 6 days (until day 47 of the protocol). At day 90, a subcutaneous rechallenge was performed, with $1 \times 10^6$ MC38 cells/mouse, in the two groups that received the highest dose of vector, and 5 naive mice were used as a control. The tumour size was measured at 13, 23 and 42 days post-rechallenge (days 103, 113 and 132 of the protocol). At day 113, the mice were bled, and the PBLs were extracted. Finally, at day 132 the animals were sacrificed and the subcutaneous tumours were extracted from those mice that had not been not fully protected. The red vertical lines indicate the days of bleeding for the measurement of serum parameters. The blue vertical lines indicate the days when the post-rechallenge tumour size was measured.

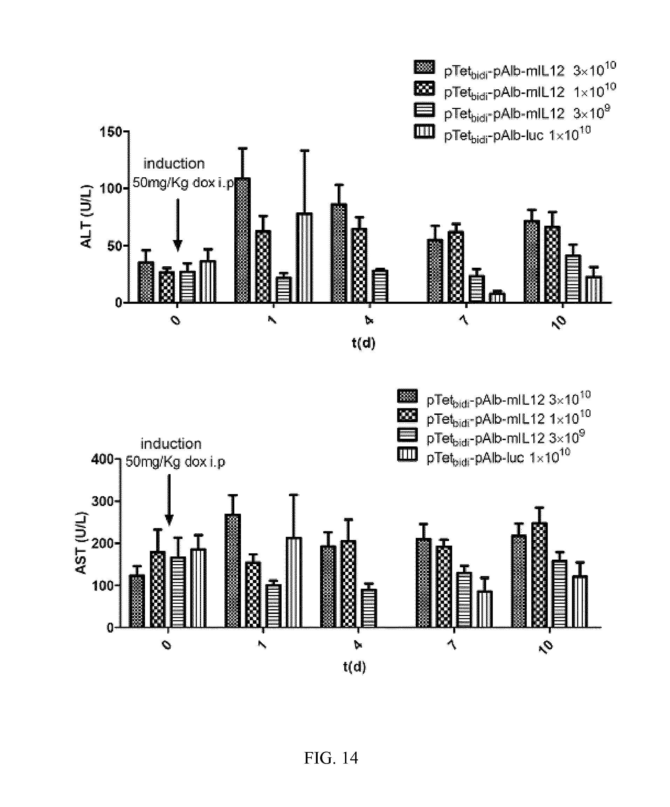

**Figure 14**. Levels of transaminases, ALT (A) and AST (B), in the serum of female C57BL/6 animals injected with AAV2/8 virions carrying rAAV-pTet$_{bidi}$-pA1b-mIL12, at three different doses: $3 \times 10^{10}$, $1 \times 10^{10}$ and $3 \times 10^9$ vg/mouse. Shown are the levels in the basal state (day 0 of induction), at days 1, 4 and 7 following an initial i.p. administration of 50 mg/kg of doxycycline, performed at day 0 of induction, followed by the administration of doxycycline in the drinking water (2 mg/ml of dox + 5% sucrose).

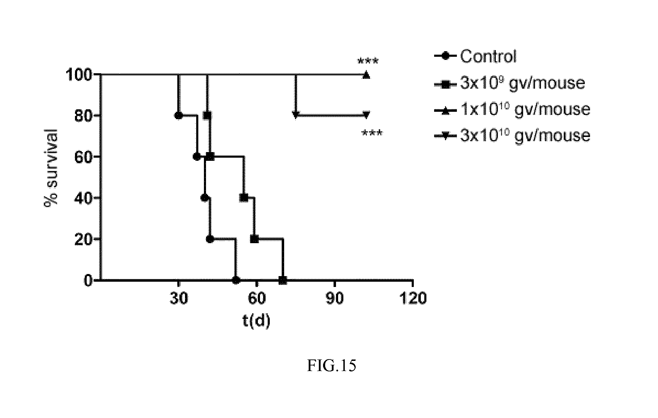

**Figure 15.** Percentage of survival in time of the C57BL/6 mice whereto the protocol described in Figure 13 was applied. The captions show the dose of virus (in vg/mouse) that each group received by intravenous route at the beginning of the protocol. The control group did not receive any vector. The statistical evaluations were performed using the Log-rank test (GraphPad Prism software) (***p<0.001).

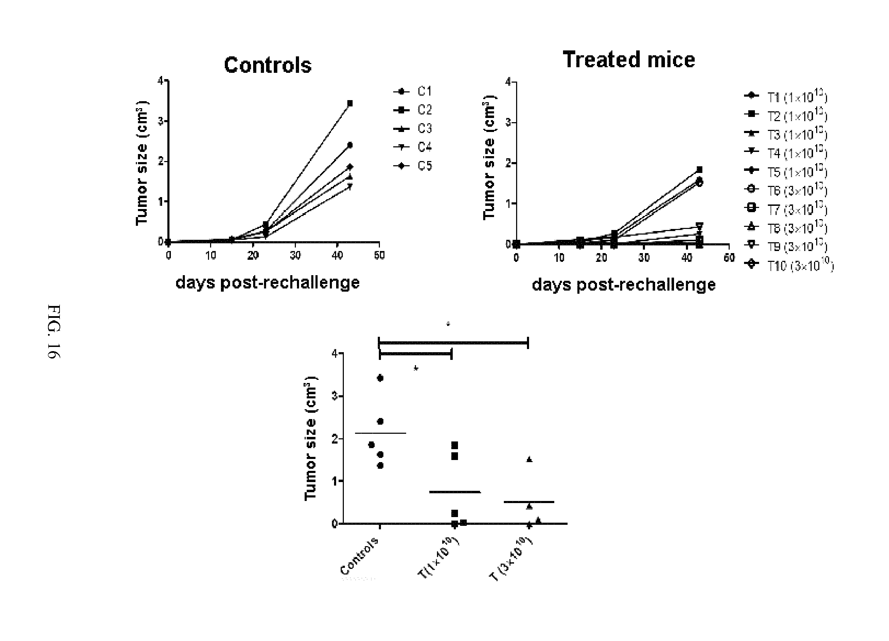

**Figure 16.** Tumour size of the treated mice that were subjected to a subcutaneous rechallenge with $1 \times 10^6$ MC38 cells/mouse (B) compared to a group of untreated mice, control (A). In parentheses (in B), the dose of virus, expressed in vg, that each mouse received in accordance with the protocol described in Figure 13, is indicated. (C) Shows the tumour sizes reached by the different groups at the end of the experiment (day 132 of the protocol). In parentheses, the dose of virus, expressed in vg, that each mouse received in accordance with the protocol described in Figure 13, is indicated.

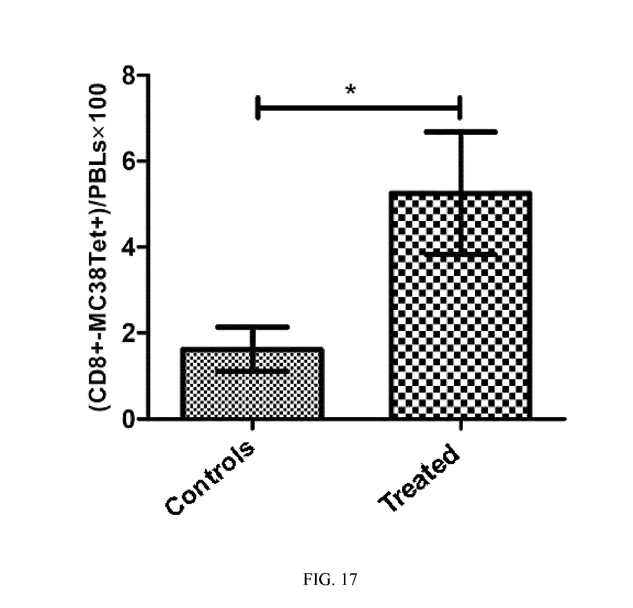

**Figure 17**. Percentage of CD8$^+$/Tet$^+$ PBLs (MC38). Blood was extracted from the mice at day 23 post-rechallenge (day 113 of the protocol described in Figure 13), the PBLs were obtained and labelled with anti-CD8+ antiboides and a tetramer loaded with an MC38-cell-specific peptide. The percentage of CD8$^+$-MC38Tet$^+$ PBLs was analysed using the FlowJo computer programme. The statistical evaluations were performed using Student's t-test (*p<0.05).

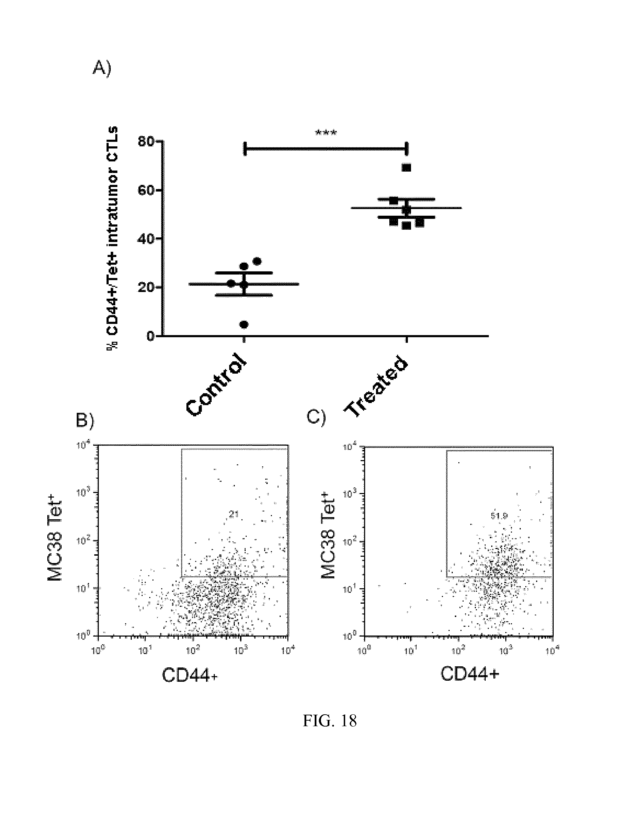

**Figure 18**. Percentage of intratumoural CD8$^+$ lymphocytes specific for the MC38 tetramer and positive for activation marker CD44. The groups of treated mice were grouped together, since there were no significant differences between them (A). B) and C) show the point diagrams pertaining to a representative mouse from the control group and one from the treated group, respectively. The statistical evaluations were performed using Student's t-test (***p<0.001).

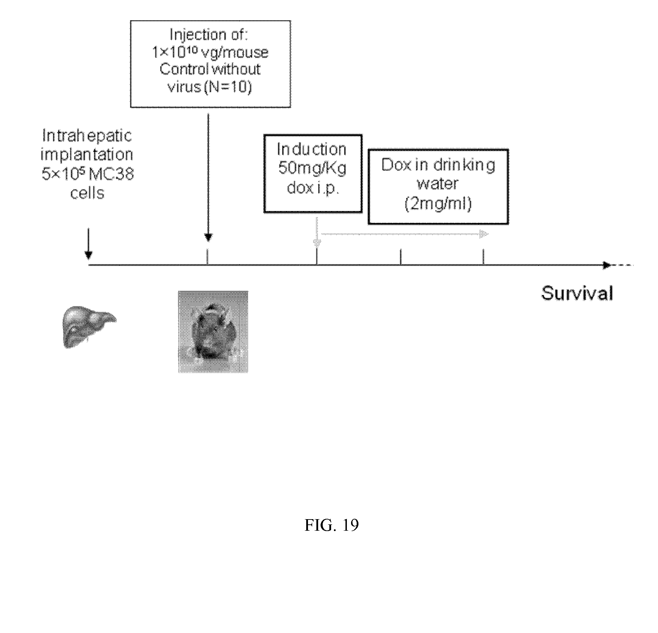

**Figure 19.** Diagram of the protocol of the therapeutic antitumor treatment administered to female C57BL/6 mice. On day 0, the cells of the syngeneic tumor line MC38 were implanted. Seven days later, a single dose of $1 \times 10^{10}$ rAAV-pTet$_{bidi}$pA1b-mIL12 viral genomes (vg) was injected into them and a group of mice was left without vector (N=5). Fifteen days later, the induction of the system was started with an (i.p) administration of doxycycline (50 mg/Kg). On the following day, the induction was continued in drinking water (2 mg/ml of dox + 5% sucrose), which was maintained for the 6 subsequent days, after which the survival in both groups was analyzed.

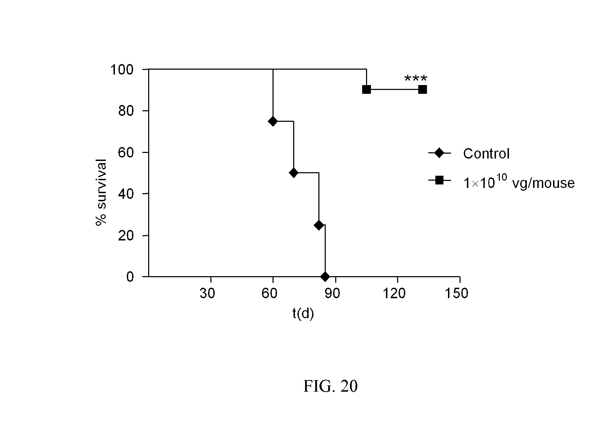

**Figure 20.** Percentage of survival over time of the C57BL/6 mice to which the protocol described in Figure 19 was applied. The legends show the dose of virus (in vg/mouse) received by the treated animals. The control group did not receive vector. The statistical evaluations were performed using the Logrank test (GraphPad Prism software) (***p<0.001).

## DETAILED DESCRIPTION OF THE INVENTION

GENE CONSTRUCTS OF THE INVENTION

The authors of this invention have developed an expression system for polynucleotides of interest which allows for a precise expression, in both temporal and spatial terms, of said polynucleotides in the liver. To this end, they use an activateable bi-directional operator-promoter that is associated to a first hepato-specific promoter which controls the expression of a transactivator that activates the expression of said bi-directional promoter in the presence of an inducer agent and to a second hepato-specific promoter which controls the expression of the gene of interest. In the basal state (not induced), the hepato-specific promoter directs the expression of small quantities of both the transactivator and the transgene, leading to the so-called residual expression of the system. In the absence of an inducer agent, the transactivator is conformationally incapable of binding to the operator sites in the bi-directional promoter and, therefore, of activating the transcription of the bi-directional promoter.

In the presence of an inducer, the latter binds to the residual transactivator molecules present in the cell, producing a conformational change that allows for it to bind to the inducible bi-directional promoter-operator and activate the transcription thereof. In this way, the expression of both the transgene and the transactivator is induced. These new synthesised transactivator molecules are capable of binding to the cell's free inducer agent and creating a positive feedback loop, until a state is reached wherein two situations may appear:

(a) that all the operator sites are occupied by inducer agent-transactivator complex molecules; or
(b) that the cell's free inducer agent is consumed, in the event that it has not been administered in excess, and that the synthesised transactivator molecules cannot continue to bind to the operator sites.

Therefore, following the administration of the inducer, the induction step or induced state will begin. The expression levels of both the transactivator and the transgene will depend on the dose of inducer agent administered. Once the inducer is withdrawn, the transactivator returns to its inactive conformational state, and is not capable of efficiently binding to the operator sites, which makes the transgene expression to decrease until it returns to the initial or basal state. The maximum expression is obtained when all the operator sites are occupied by inducer agent-transactivator complex molecules.

The authors of this invention have shown that, surprisingly, the vectors developed allow for a hepato-specific expression following the induction that reaches higher levels than those obtained using vectors with promoters with a higher basal expression. Thus, although the maximum expression of the induction systems described thus far is directly correlated with the potency of the promoter in the basal state, in the system of this invention, which uses tissue-specific promoters that are generally weaker than the ubiquitous CMV-type promoters, a higher basal expression following the induction is obtained than with CMV. Specifically, in example 2 of this invention, it may be observed that the rate of induction of a reporter gene obtained using the hepato-specific system of the invention following the administration of the inducer agent is approximately 85 times greater than the rate of induction of the system based on the ubiquitous CMV promoter (see figure 7), which disagrees with the results obtained by Zabala et al. (Zabala, M., et al., Cancer Res. 2004; 64: 2799-2804) wherein the use of a Palb promoter to direct the expression of a transgene resulted in a lower expression of the transgene in comparison to that obtained using a system wherein a cytomegalovirus minimal promoter was used. Moreover, the difference between both systems in the induced state for this dose of dox is highly significant. On the other hand, the expression in liver of a reporter gene controlled by the inducible hepato-specific expression system of this invention reaches higher luciferase activity induction levels than the system based on the ubiquitous CMV promoter (see figure 11).

Thus, a first aspect of the invention relates to a gene construct that allows for the inducible hepato-specific expression of a polynucleotide of interest in response to an inducer agent, which comprises

(i) an inducible bi-directional operator-promoter that comprises a responsive element to said inducer agent flanked by a first hepato-specific promoter sequence and a second hepato-specific promoter sequence, wherein both hepato-specific promoter sequences are oriented in a divergent manner,
(ii) a first nucleotide sequence that comprises a sequence which encodes a transactivator that may be activated by said inducer agent and a polyadenylation signal located at the 3' position with respect to the region that encodes the transactivator, wherein the sequence that encodes an activateable transactivator is operatively coupled to the first hepato-specific promoter sequence, and
(iii) a second nucleotide sequence that comprises a polynucleotide which is operatively coupled to the second hepato-specific promoter sequence and a polyadenylation signal located at the 3' position with respect to the polynucleotide of interest,

wherein the promoter activity of said first and second hepato-specific promoter sequences is induced as a consequence of the binding of the transactivator to the operator region of the operator-promoter in the presence of the inducer agent.

The expression "gene construct", as used in this invention, refers to a single-chain or double-chain nucleic acid, which comprises a region capable of being expressed and, optionally, regulatory sequences that precede said nucleic acid (non-encoding 5'-sequences) or follow said nucleic acid (non-encoding 3'-sequences). The expressions "gene construct" and "nucleic acid construct" are used interchangeably in this invention.

The term "expression" refers to the transcription of a gene or genes, or a gene construct, to produce structural RNA (rRNA, tRNA) or mRNA, with or without the subsequent translation of said RNA into a protein.

The expression "inducible expression", as used in this invention, refers to the fact that the expression may increase in response to an activator/inducer.

The expression "polynucleotide of interest", as used in this invention, refers to a nucleic acid sequence that is partially or totally heterologous with respect to the cell or subject wherein it is introduced and which, due to the presence of expression regulatory regions at positions 5' or 3' with respect to said polynucleotide of interest, may be transcribed and, eventually, translated in order to produce a polypeptide with a desired biological activity. The expression "polynucleotide of interest" should not be solely understood to mean a polynucleotide with the capacity to encode a polypeptide, but may also be used to refer to a nucleic acid sequence that is partially or totally complementary to an endogenous polynucleotide of the cell or subject wherein it is to be introduced, such that, following the transcription thereof, it generates an RNA molecule (microRNA, shRNA or siRNA) capable of hibridising and inhibiting the expression of the endogenous polynucleotide. The polynucleotide of interest may be DNA or cDNA.

The expression "inducer agent", as used in this invention, refers to any molecule that is capable of causing an increase in the transcription of a gene. Usually, the gene the transcription whereof is induced in response to said inducer agent is under the operative control of a transcription regulatory region which, in turn, has binding sites for a transcription activator the activity whereof increases in the presence of said inducer agent. Thus, in the context of this invention, since it refers to the transcription regulatory region of a gene of interest, the expression "responsive element to the inducer agent" is used to refer to the binding sites for a transcription activator the activity whereof increases by the binding of the inducer agent. Preferably, the inducer agent is a compound that is easy to administer and easily distributed in the body, and innocuous at the doses used to activate the system. Moreover, it must be capable of penetrating into the desired tissue or organ, and have a half-life of several hours (not minutes or days).

Regulatable bi-directional operator-promoter

Element (a) of the gene construct of the invention comprises an inducible bi-directional operator-promoter that comprises a responsive element to a transactivator in its active form, flanked by a first hepato-specific promoter sequence and a second hepato-specific promoter sequence, wherein both hepato-specific promoter sequences are oriented in a divergent manner.

The expression regulatable bi-directional operator-promoter", as used in this invention, refers to a promoter that is capable of activating the transcription of specific polynucleotides in opposite directions from said "operator-promoter" in the presence of a given signal.

The term "responsive element to an inducer agent" refers to one or more DNA elements that act in cis and which confer to a promoter the capacity to activate the transcription in response to the interaction of said element with the DNA binding domains of a transcription factor or a transactivator the transcriptional activity whereof is induced in the presence of the inducer agent, normally due to a conformational change in the transactivator resulting from the binding to the inducer agent. Therefore, the expression "responsive element to an inducer agent" must be understood to mean a responsive element to a transcription activator in the presence of an inducer agent. The DNA binding domain of the transcription factor or transactivator is capable of binding, in the presence or absence of the activator agent, to the DNA sequence of the responsive element in order to initiate or inhibit the transcription of genes located at the 3' position with respect to the promoter. The term "responsive element" is used interchangeably with "trancriptional responsive element" or TRE.

In a preferred form of embodiment, the regulatable bi-directional promoter-operator comprises at least one responsive element to a transactivator that may activated by antibiotics, preferably a tetracycline-responsive element and, even more preferably, a tetracycline-responsive element that comprises a variable number of copies of the 42-base-pair operator sequence (called TetO), as originally described in Baron et al. (Nucleic Acids Res., 1995, 17: 3605-3606). The number of copies of TetO may be at least 2, at least 5 or, preferably, no more than 7. This type of tetracycline-responsive elements may activate bi-directional transcription in the presence of the reverse tetracycline-activated transactivator (or its analogue doxycycline), as originally described by Gossen et al. (Science, 1995, 278: 1766-1769). In a preferred form of embodiment, the responsive element to transactivator + tetracycline comprises 7 copies of the operator sequence, in which case it is called TetO7. In an even more preferred form, the operator-promoter comprises or consists of sequence SEQ ID NO: 1.

Element (a) of the gene construct additionally comprises a first and a hepato-specific promoter sequence.

The expression "transcription promoter sequence", as used in this invention, refers to a nucleic acid sequence that is recognised by a host cell and results in the activation of the transcription of nucleic acid sequences present at the 3' position with respect to said promoter region. Generally, the promoter sequence contains transcription control sequences that allow for the expression of the polynucleotide of interest.

The expression "hepato-specific", as used in this invention to refer to the transcription promoter region, refers to the fact that said region is capable of selectively activating transcription in hepatic cells or in cell lines derived from hepatic cells. Liver-specific promoters suitable for this invention include, without limitation, the promoter of $\alpha$-1-antitrypsin (AAT), the promoter of thyroid-hormone-binding globulin, the promoter of alpha-fetoprotein, the promoter of alcohol dehydrogenase, the promoter of IGF-II, the promoter of factor VIII (FVIII), the promoter of HBV Basic Core Protein or BCP and the PreS2 promoter, the promoter of thyroxine-binding globulin (TBG), the hybrid promoter of the hepatic control region (HCR)-ApoCII, the HCR-hAAT hybrid promoter, the AAT promoter combined with the enhancer element of the mouse albumin gene (Ealb), the promoter of apolipoprotein E, the promoter of low-density lipoprotein, the promoter of pyruvate kinase, the promoter of phosphenol pyruvate carboxykinase, the promoter of lecithin-cholesterol acyl transferase (LCAT), the promoter of apolipoprotein H (ApoH), the promoter of transferrin, the promoter of transthyretin, the promoters of alpha-fibrinogen and beta-fibrinogen, the promoter of alpha-1-antichymotrypsin, the promoter of alpha-2-HS glycoprotein, the promoter of haptoglobin, the promoter of ceruloplasmin, the promoter of plasminogen, promoters of the complement proteins (CIq, CIr, C2, C3, C4, C5, C6, C8, C9, factor I and factor H of the complement), the promoter of the complement C3 activator, the promoter of hemopexin and the promoter of the $\alpha$-1-acid glycoprotein. Additional tissue-specific promoters may be found in the Tissue-Specific Promoter Database, TiProD (Nucleic Acids Research, J4:D104-D107 (2006)). Alternatively, it is possible to use hybrid promoters that comprise a liver-specific enhancer and a liver-specific promoter. This type of promoters include the hybrid promoter of the hepatic control region (HCR)-ApoCII, the HCR-hAAT hybrid promoter, the AAT promoter combined with the enhancer element of the mouse albumin gene (Ealb) and a promoter of apolipoprotein E, the hybrid promoter formed by the enhancer of the mouse albumin gene (Ealb) and the promoter of mouse alpha-1-anti-trypsin (AAT) (Ealb-AATp).

In a preferred form of embodiment, the hepato-specific promoter that is a part of the first expression cassette is the albumin gene promoter of murine origin or human origin. In particular, this invention considers the use of the full albumin gene promoter (SEQ ID NO: 2) or the minimal region of said promoter (SEQ ID NO: 3), corresponding to nucleotides 113 to 196 of the full promoter defined in SEQ ID NO: 2. The invention considers the use of any fragment of the promoter that includes, at least, the minimal promoter (residues 113-196 of SEQ ID NO: 2).

In the context of this invention, a liver-specific promoter is a promoter that is more active in the liver as compared to its activity in any other body tissue. Typically, the activity of a liver-specific promoter will be considerably greater in the liver than in other tissues. For example, such promoter may be at least 2, at least 3, at least 4, at least 5 or at least 10 times more active in hepatic tissue than in other types of cells. The activity of said promoter in cells of hepatic origin as compared to a reference cell may be determined by its capacity to direct the expression in a given tissue whilst preventing the expression in other cells or tissues. As a result, a liver-specific promoter allows for an active expression of the gene bound in the liver and prevents the expression in other cells or tissues.

Those skilled in the art will note that the first and the second hepato-specific transcription promoter regions may be identical or may be different. In a preferred form of embodiment, both transcription regulatory regions are identical. In an even more preferred form of embodiment, both the first transcription promoter region and the second transcription promoter region comprise the albumin gene promoter. In an even more preferred form of embodiment, the albumin gene promoter that forms the first and/or second transcription promoter region comprises a sequence selected from the group of SEQ ID NO: 2 and SEQ ID NO: 3.

In a preferred form of embodiment, the regulatable bi-directional promoter-operator comprises a tetracycline-responsive element formed by seven binding sites to the transactivator that may be activated by the inducer agent, preferably tetracycline, which is flanked by two albumin gene promoters oriented in a divergent manner. In an even more preferred form of embodiment, said operator-promoter that comprises the TetO7 operator and two albumin promoters comprises sequence SEQ ID NO: 4.

The sequences with hepato-specific promoter activity are oriented in a divergent manner with respect to the operator region. The expression "divergent orientation", as used in this invention to refer to hepato-specific promoters, refers to pairs of promoters wherein the activation of the transcription mediated by the first promoter of the pair takes place on one of the DNA molecule strands, thereby allowing for RNA polymerase to act in the 5'-3' direction, whereas the second promoter would act on the opposite strand, leading to the displacement of RNA polymerase in the opposite direction to that wherein it acts jointly with the first promoter.

The elements that form the bi-directional operator-promoter of the gene construct of the invention are organised in such a way that the promoter activity of the first and the second hepato-specific promoter sequences is induced as a consequence of the binding of the transactivator to the operator region of the operator-promoter in the presence of the inducer agent. Those skilled in the art will note that said induction of the transcription promoter activity will depend, amongst other factors, on the distance between the operator element and the hepato-specific promoter sequences. Preferably, the distance between the operator and the first or second promoter sequences may vary between 0 and 30 nucleotides, more preferably between 0 and 20, and, even more preferably, between 0 and 15 nucleotides.

First nucleotide sequence of the gene construct of the invention

Element (b) of the gene construct of the invention is a nucleotide sequence that comprises: a sequence that encodes a transactivator which may be activated by said inducer agent that is operatively coupled to the first hepato-specific promoter sequence; and a polyadenylation signal located at the 3' position with respect to the region that encodes the transactivator.

The expressions "nucleotide sequence", "nucleic acid" and "polynucleotide" are used interchangeably in this invention to refer to the polymer form of phosphate esters of ribonucleosides (adenosine, guanosine, uridine or cytidine; "RNA molecules") or deoxyribonucleosides (deoxyadenosine, deoxyguanosine, deoxythymidine or deoxycytidine; "DNA molecules"), or any analogous phosphoester thereof, such as phosphorothioates and thioesters, in single-strand or double-strand form. Thus, helices formed by DNA-DNA, DNA-RNA and RNA-RNA are possible. The term "nucleic acid sequence" and, in particular, DNA or RNA molecule, refers solely to the primary or secondary structure of the molecule and does not limit any particular type of tertiary structure. Thus, this term includes double-chain DNA as it appears in linear or circular DNA molecules, supercoiled DNA plasmids and chromosomes.

The expression "transactivator that may be activated by the inducer agent", as used in this invention, refers to a polypeptide that, when bound to said inducer agent, is capable of promoting the transcription of a given gene by binding to specific recognition regions for said polypeptide in the non-encoding region of said gene, that is, its activity may be modulated by additional factors which may be supplied or eliminated depending on the need to promote the transcription of the genes that comprise specific binding sites for said regulators. Those skilled in the art will note that the transactivator encoded by the first nucleotide sequence of the invention is capable of binding to the region of the operator-promoter that comprises the responsive elements to said inducer agent, such that the transactivator activates the transcription of said first and second hepato-specific promoter sequences following the binding thereof to the operator region of the operator-promoter in the presence of the inducer agent.

Additionally, those skilled in the art will note that the invention considers any method of regulating the expression of the transcription regulator, provided that it allows for regulated expression with a minimal basal transcription. In particular, the invention considers the use of transcription regulators the induction whereof takes place not by an increase in the expression levels of the transcription regulator, but by means of a conformational change in response to the binding of the inducer agent, which may lead to the translocation of the transcription factor to the nucleus, where it exerts its effect, or an increase in the transcriptional activity. This type of transcription regulators are usually composed of a DNA binding domain, or DBD, a ligand binding domain, or LBD, and a transcription activation domain, or AD.

The DNA binding domain may be any domain for which there is a known specific binding element, including synthetic, chimeric or analogous DNA binding domains. DNA binding domains suitable for this invention include: (i) homeodomains (Scott et al., 1989, Biochim. Biophys. Acta 989: 25-48; Rosenfeld et al., 1991, Genes Dev. 5: 897-907), which are generally composed of a chain of approximately 61 amino acids that presents a secondary structure composed of three alpha helices, (ii) zinc fingers formed by two to three dozen DNA fingers with the general formula $Cys_2His_2$, organised in tandem (for example, TFIIIA, Zif268, Gli and SRE-ZBP), wherein each module comprises an alpha helix capable of coming in contact with a DNA region of 3 to 5 base pairs, at least 3 zinc fingers being necessary to generate a high-affinity DNA binding site, and at least two zinc fingers being necessary to generate low-affinity DNA binding sites, (iii) the DNA binding domains called helix-turn-helix, or HLH, such as TetR, MAT1, MAT2, MATa1, Antennapedia, Ultrabithorax, Engrailed, Paired, Fushi tarazu, HOX, Unc86, Oct1, Oct2 and Pit-1, (iv) DNA binding domains of the leucine zipper type, such as GCN4, C/EBP, c-Fos/c-Jun and JunB. Examples of DNA binding domains suitable for this invention include the DNA binding domain of GAL4, LexA, transcription factors, group H nuclear receptors, nuclear receptors of the steroid/thyroid hormone superfamily. Those skilled in the art will note that the invention considers the use of hybrid DNA binding domains composed of several DNA binding motifs that may recognise DNA binding sites different from those of the elements that compose them. Thus, it is possible to use DNA binding domains formed by the binding of a zinc finger and a homeobox. In a preferred form of embodiment, the DNA binding domain is that obtained from the Tet transcription repressor of *E.coli.*

The ligand binding sequences capable of promoting the nuclear localisation of a transcription activator that contains them suitable to be used in this invention include the PPAR-derived localisation sequence (receptors activated by peroxisomal activators), which are translocated to the nucleus in the presence of 15-deoxy-[Delta]-prostaglandin J2, retinoic acid receptors, which are translocated to the nucleus in the presence of the alpha, beta or gamma isomers of 9-cis-retinoic acid, receptors of farnesoid X, which may be activated by retinoic acid and TTNPB, hepatic X receptors, which may be activated by 24-hydroxycholesterol, benzoate X receptors which may be activated by 4-amino-butylbenzoate, constitutive androstane receptor, pregnan receptors, which may be induced by pregnelone-16-carbonitrile, receptors of steroids and xenobiotics, which may be induced by rifampycin, progesterone receptors, which may be activated by medroxyprogesterone, as well as agonists and antagonists of mifepristone and derivatives of 19-nortestosterone, receptors of glucocorticoids, which may be activated by glucocorticoids, thyroid hormone receptors, which may be activated by T3 and/or T4, and oestrogen receptors, which may be activated by oestrogens and the derivatives thereof, such as 17-beta-estradiol and estradiol, tTA transactivators, which may be activated by "tet-off" tetracycline/doxycycline (Gossen and Bujard, 1992, Proc. Natl. Acad. Sci. USA, 89: 5547-5551), rtTA transactivators, which may be activated by "tet-on" tetracyclines (Gossen et al., 1995, Science, 268: 1766-1769), transactivators which may be induced by muristerone A or ligands analogous to the ecdysone receptor (No et al., 1996, Proc. Natl. Acad. Sci. USA, 93: 3346-3351), transactivators which may be activated by the RSL1 ligand, such as the RheoSwitch system initially described by Palli et al. (2003, Eur. J. Biochem., 270: 1308-1315), a transactivator which may be activated by rapamycin or analogues of rapamycin (Ho et al., 1996, Nature, 382: 822-826; Amara et al., 1997, Proc. Natl. Acad. Sci. USA, 94: 10618-10623), and a transactivator that may be activated by coumermycin/novobiocin, which act competitively as inducer and repressor, respectively (Zhao et al., 2003, Hum. Gene Ther., 14: 1619-1629).

Finally, the transcription activation domain may be an acidic activation domain, a proline-rich activation domain, a serine/threonine-rich activation domain and a glutamine-rich activation domain. Examples of acidic activation domains include the VP16 regions and the GAL4 region formed by amino acids 753-881. Examples of proline-rich transcription activation domains include amino acids 399-499 of CTF/NF1 and amino acids 31-76 of AP2. Examples of serine/threonine-rich activation domains include amino acids 1-427 of ITF1 and amino acids 2-452 of ITF2. Examples of glutamine-rich activation domains include amino acids 175-269 of Oct1 and amino acids 132-243 of Sp1. The sequences of each of the regions described, as well as other transcription activation domains, have been described by Seipel, K. et al. (EMBO J. (1992) 13: 4961-4968). Additionally, other transcription activation domains may be obtained from those mentioned above using methods known in the state of the art. Additionally, the activation domain may be the activation domain of group H nuclear receptors, of the nuclear receptors of thyroid or steroid hormones, the activation domain of VP16, of GAL4, of NF-κB, of B42, of BP64, or of p65.

In a preferred form of embodiment, the transcription activation domain is protein 16 of the herpes simplex virion (hereinafter VP16), the amino acid sequence whereof has been described by Triezenberg, S. J., et al. (Genes Dev., 1988, 2: 718-729). This domain may be formed by about 127 amino acids of the C-terminal end of VP16. Alternatively, the transcription activation domain may be formed by the 11 amino acids of the C-terminal region of VP16, which maintain the capacity to activate the transcription. Regions of the C-terminal end of VP16 suitable to be used as transcription activation domains have been described by Seipel, K. et al. (EMBO J. (1992) 13: 4961-4968). In an even more preferred form of embodiment, the transcription activator comprises the minimal region of said protein formed by 13 amino acids with the sequence PADALDDFDLDML (SEQ ID NO: 5), as described by Baron et al. (Nucleics Acids. Res., 1997, 25: 2723-2729).

In a preferred form of embodiment, the transcription activator is a transcription activator which may be activated by tetracycline or the analogues thereof.

The term "tetracycline analogue", as used in this invention, refers to compounds that are structurally related to tetracycline, which have the capacity to bind to the tetracycline repressor (TetR) with a Ka of at least about $10^{-6}$ M$^{-1}$. Preferably, the tetracycline analogue has an affinity for TetR of at least $10^{-9}$ M$^{-1}$. Examples of tetracycline analogues suitable for this invention include, without limitation, anhydrotetracycline, doxycycline (Dox), chlorotetracycline, oxytetracycline, epioxytetracycline, cyanotetracycline, demeclocycline, meclocycline, metacycline and others which have been described by Hlavka and Boothe, "The Tetracyclines", in "Handbook of Experimental Pharmacology" 78, R. K. Blackwood et al. (eds.), Springer-Verlag, Berlin-New York, 1985; L. A. Mitscher, "The Chemistry of the Tetracycline Antibiotics", Medicinal Research 9, Dekker, N.Y., 1978; Noyee Development Corporation, "Tetracycline Manufacturing Processes", Chemical Process Reviews, Park Ridge, N.J., 2 volumes, 1969; R. C. Evans, "The Technology of the Tetracyclines", Biochemical Reference Series 1, Quadrangle Press, New York, 1968; and H. F. Dowling, "Tetracycline", Antibiotic Monographs, no. 3, Medical Encyclopedia, New York, 1955.

In a preferred form of embodiment, the transactivator that may be activated by tetracyclines may be the so-called reverse tetracycline repressor protein, or reverse tetR, which refers to a polypeptide that (i) shows specific affinity for the inducer agent, (ii) shows specific affinity for the tet-type responsive element when it is bound to the inducer agent and (iii) is displaced from the tet element when it is not bound to the inducer agent. This activator includes both natural forms and functional derivatives thereof. In a preferred form of embodiment, the activator that may be regulated by tetracyclines may be the so-called reverse tetracycline-dependent transactivator (rtTA), characterised in that, in the presence of tetracycline or the analogues thereof, it undergoes a conformational change that allows for it to become a transcription activator, whilst being inactive in the absence of tetracycline. In this way, the problems associated with transactivators derived from the tetR repressor of *E.coli,* which are capable of activating the transcription of genes that present tetracycline-responsive elements in the absence of tetracycline and which, in the presence of tetracycline, would cease to activate them, are prevented. Reverse tetracycline-dependent transactivators (rtTA) include, preferably, the rtTA transactivator or any of the variants of rtTA described by Urlinger, S., et al. (Proc. Natl. Acad. Sci USA, 2000; 97: 7963-7968). In a preferred form of embodiment, the variant of rtTA is the variant known as rtTA-M2, characterised in that, in order to be activated, it requires a concentration of doxycycline that is 10 times lower than that required by the original rtTA. The rtTA-M2 transactivator is a polypeptide encoded by the polynucleotide with sequence SEQ ID NO: 6.

Element (b) of the first nucleotide sequence of the gene construct of the invention additionally comprises a polyadenylation sequence that is located at the 3' position with respect to the polynucleotide that encodes the transactivator. The expression "polyadenylation sequence" or "polyadenylation signal", as used in this invention, refers to a nucleic acid that contains a transcription termination signal and which, when it appears in an RNA transcript, allow for said transcript to be polyadenylated in the presence of an enzyme with polyadenyl transferase activity. "Polyadenylation", as used herein, refers to the addition of a polyadenine stretch to the 3'-end of mRNA. Polyadenylation signals suitable to be used in this invention include, without limitation, the SV40 early-late polyadenylation signal, the polyadenylation signal of HSV thymidine kinase, the polyadenylation signal of the protamine gene, the polyadenylation signal of adenovirus 5 Elb, the polyadenylation signal of the bovine growth hormone, the polyadenylation signal of the human variant of the growth hormone and similar ones.

In a preferred form of embodiment, the polyadenylation signal is a bi-directional polyadenylation signal. The use of a bi-directional polyadenylation signal is particularly advantageous when the gene construct of the invention is to be expressed using viral vectors wherein the termination sequences have a certain transcription promoter activity (in particular AAVs, lentiviruses). In this way, it is prevented from interfering with the inducible system, thereby reducing the basal activity. In an even more preferred form of embodiment, the bi-directional polyadenylation signal corresponds to the SV40 polyadenylation signal. In an even more preferred form of embodiment, the SV40 polyadenylation signal comprises sequence SEQ ID NO: 7.

Second nucleotide sequence of the gene construct of the invention

The gene construct of the invention additionally comprises a polynucleotide that is operatively coupled to the second hepato-specific promoter sequence and a polyadenylation signal located at the 3' position with respect to the polynucleotide of interest.

The terms "nucleotide sequence", "polynucleotide", "hepato-specific promoter sequence", "operative control" and "polyadenylation signal" have been defined above and are used in the first nucleotide sequence in the same way as in the first nucleotide expression sequence. The term "polynucleotide of interest", as used in this invention, refers to a DNA sequence the manipulation whereof is desireable for different reasons and which includes DNA, cDNA, genomic DNA, RNA or analogues of nucleic acids, as well as the corresponding anti-sense molecules that are capable of generating a protein or an RNA molecule, such as, for example, in a non-limiting manner, small interfering RNA (siRNA), short-loop RNA (shRNA) or ribozymes.

In a preferred form of embodiment, the polynucleotide of interest encodes a polypeptide. This polypeptide may be a gene of the luciferase reporter gene type, green fluorescent protein (GFP), variants of GFP (EGFP, YFP or BFP), alkaline phosphatase, beta-galactosidase, beta-glucuronidase, catechol dehydrogenase.

Alternatively, the polynucleotide of interest encodes a polypeptide the expression whereof in the liver or in hepatic cells is useful for the correction of hepatic disorders that will benefit from the expression of said polypeptide. Thus, polypeptides suitable to be used in the treatment of hepatic alterations include, without limitation, an interferon a and, specifically, an IFN-$\alpha$ selected from the group formed by IFN-$\alpha$2a, IFN-$\alpha$2b, IFN-$\alpha$4, IFN-$\alpha$5, IFN-$\alpha$8, oncostatin, cardiotrophin, IL-6, IGF-I and variants thereof, amphiregulin, IL-15, IL-12, CD134, CD137, PBGD, antibodies, TGF-$\beta$1 inhibitors, such as peptides P17 and P144 described in international patent applications WO0031135, WO200519244 and WO0393293, which are incorporated herein by reference thereto, IL-10 inhibitors, FoxP3 inhibitors, TNF$\alpha$ inhibitors, VEGF inhibitors, PD-1 inhibitors and CD152 inhibitors.

In a preferred form of embodiment, the polynucleotide of interest encodes IL-12 or a functionally equivalent variant thereof. Interleukin-12 (IL-12) is a type I cytokine that is primarily secreted by macrophages and dendritic cells, includes both native IL-12 and IL-12 prepared in a recombinant manner, and is capable of increasing anti-tumour immunity through numerous mechanisms, which include: (1) increase in the responses of cytotoxic T lymphocytes, (2) activation of natural cytolytic cells (NK, natural killer), (3) enhancement of the proliferation of natural cytolytic cells and T lymphocytes, (4) induction of the polarisation of a sub-series of helper T cells type I (Th1, T helper 1), and (5) induction of an anti-angiogenic effect. Many of these activities are mediated by the production and secretion of interferon-$\gamma$ (INF-$\gamma$) by natural cytolytic cells and activated T lymphocytes.

Cytokine IL-12 is a heterodimer that is composed of a heavy chain (p40) and a light chain (p35). The sequences of the light and heavy chains of human origin have been described by Gubler et al. (Proceedings of the National Academy of Sciences, USA, 1991, 88: 4143). The polynucleotide of interest may encode the heavy chain if the light chain is exogenously supplied, it may encode the light chain if the heavy chain is exogenously supplied, or it may encode both chains. The polynucleotide that encodes IL-12 produces a single RNA that comprises two open reading frames separated by an internal ribosome entry site, which leads to the expression of each of the chains from each of the open reading frames. Preferably, the polynucleotide that encodes IL-12 comprises a single open reading frame that encodes a fusion protein which comprises the light chains and the heavy chains bound to one another by a linker, as described in WO9624676 and in Lieschke G.J., et al. (Nat Biotechnol. 1997, 15: 35-40). In a preferred form, the polynucleotide that encodes single-chain IL-12 comprises sequence SEQ ID NO: 8.

The term "functionally equivalent variant", as used in this invention, refers to polypeptides that differ from the sequence of IL-12 by one or more insertions, deletions or substitutions, but which substantially maintain the biological activity of IL-12. The functionally equivalent variants of IL-12 suitable to be used in this invention present a sequence identity with said cytokine of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity between the variants and the immunostimulating cytokines is determined using computer algorithms and methods that are widely known to those skilled in the art. The identity between two amino acid sequences is preferably determined using the BLASTP algorithm [BLASTManual, Altschul, S., et al., NCBI NLM NIH Bethesda, Md. 20894, Altschul, S., et al., J Mol Biol, 215: 403-410 (1990)]. The functions of IL-12 that may be monitored to determine whether a given polypeptide is a functionally equivalent variant of IL-12 include, without limitation, differentiation of immature T cells in Th1 cells, stimulation of the growth and function of T cells, synthesis of IFN-$\gamma$ and TNF-$\alpha$ by NK (natural killer) cells, reduction in the IL-4-mediated suppression of IFN-$\gamma$, increase in the cytotoxic activity ofNK cells and CD8+ lymphocytes, stimulation of the expression of the beta 1 and beta 2 chains of the IL-12 receptor and anti-angiogenic activity. Preferably, the IL-12 activity of a variant is determined by measuring the capacity to increase anti-tumour immunity, as determined, for example, by means of the assay described by Zabala, M., et al., 2007 [J Hepatology, vol. 47(6): 807-815].

## VECTORS, VIRAL GENOMES AND VIRIONS OF THE INVENTION

The gene construct of the invention may be presented in isolated form. However, in order to facilitate the manipulation and propagation thereof, it is convenient to incorporate the construct into a vector. Thus, another aspect of the invention relates to a vector that comprises a gene construct of the invention.

As used in this invention, the term "vector" refers to a vehicle whereby a polynucleotide or a DNA molecule may be manipulated or introduced into a cell. The vector may be a linear or circular polynucleotide, or it may be a larger-size polynucleotide or any other type of construct, such as DNA or RNA from a viral genome, a virion or any other biological construct that allows for the manipulation of DNA or the introduction thereof into the cell. It is understood that the expressions "recombinant vector" and "recombinant system" may be used interchangeably with the term "vector". Those skilled in the art will note that there is no limitation in terms of the type of vector that may be used, since said vector may be a cloning vector suitable for propagation and to obtain the adequate polynucleotides or gene constructs or expression vectors in different heterologous organisms suitable for the purification of the conjugates. Thus, suitable vectors in accordance with this invention include expression vectors in prokaryotes, such as pUC18, pUC19, Bluescript and the derivatives thereof, mp18, mp19, pBR322, pMB9, ColE1, pCR1, RP4, phages and "shuttle" vectors, such as pSA3 and pAT28, expression vectors in yeasts, such as vectors of the 2-micron plasmid type, integration plasmids, YEP vectors, centromere plasmids and similar ones, expression vectors in insect cells, such as the vectors in the pAC series and the pVL series, expression vectors in plants, such as vectors from the pIBI, pEarleyGate, pAVA, pCAMBIA, pGSA, pGWB, pMDC, pMY, pORE series and similar ones, and expression vectors in higher eukaryotic cells based on viral vectors (adenoviruses, viruses associated with adenoviruses, as well as retroviruses and lentiviruses) and non-viral vectors, such as pSilencer 4.1-CMV (Ambion), pcDNA3, pcDNA3.1/hyg, pHCMV/Zeo, pCR3.1, pEFl/His, pIND/GS, pRc/HCMV2, pSV40/Zeo2, pTRACER-HCMV, pUB6/VS-His, pVAX1, pZeoSV2, pCI, pSVL and pKSV-10, pBPV-1, pML2d and pTDT1.

The vector of the invention may be used to transform, transfect or infect cells susceptible to being tranformed, transfected or infected by said vector. Said cells may be prokaryotic or eukaryotic. As an example, the vector wherein said DNA sequence is introduced may be a plasmid or a vector which, when introduced into a host cell, is integrated into the genome of said cell and replicates jointly with the chromosome (or chromosomes) wherein it has become integrated. The obtainment of said vector may be performed by conventional methods known to those skilled in the art (Sambrook et al., 2001, cited *supra).*

Therefore, another aspect of the invention relates to a cell that comprises a polynucleotide, a gene construct or a vector of the invention; to this end, said cell has been transformed, transfected or infected with a construct or vector provided by this invention. Transformed, transfected or infected cells may be obtained by conventional methods known to those skilled in the art (Sambrook et al., 2001, cited *supra).* In a particular embodiment, said host cell is an animal cell transfected or infected with an appropriate vector.

Host cells suitable for the expression of the conjugates of the invention include, without being limited thereto, cells from mammals, plants, insects, fungi and bacteria. Bacterial cells include, without being limited thereto, cells from Gram-positive bacteria, such as species from the genera Bacillus, Streptomyces and Staphylococcus, and cells from Gram-negative bacteria, such as cells from the genera Escherichia and Pseudomonas. Fungi cells preferably include cells from yeasts such as *Saccharomyces, Pichia pastoris* and *Hansenula polymorpha.* Insect cells include, without limitation, Drosophila cells and Sf9 cells. Plant cells include, amongst others, cells from cultivated plants, such as cereals, medicinal plants, ornamental plants or bulbs. Mammalian cells suitable for this invention include epithelial cell lines (porcine, etc.), osteosarcoma cell lines (human, etc.), neuroblastoma cell lines (human, etc.), epithelial carcinomas (human, etc.), glial cells (murine, etc.), hepatic cell lines (from monkeys, etc.), CHO (Chinese Hamster Ovary) cells, COS cells, BHK cells, HeLa, 911, AT1080, A549, 293 or PER.C6 cells, human NTERA-2 ECC cells, D3 cells from the mESC line, human embryonary stem cells, such as HS293 and BGV01, SHEF1, SHEF2 and HS181, NIH3T3, 293T, REH and MCF-7 cells, and hMSC cells.

Alternatively, the gene construct of the invention may be a part of a recombinant viral genome. Thus, in another form of embodiment, the invention relates to a recombinant viral genome that comprises a gene construct in accordance with the invention. The term "viral genome", as used in this invention, refers to the genetic complement of a virus, whether complete or manipulated in such a way that the non-essential elements have been eliminated and the essential elements have been preserved, thereby maintaining the adequate functionality to infect, transduce and introduce a sequence of nucleotides of interest into a target cell.

In a preferred form of embodiment, the viral genome that comprises the construct of the invention is the genome of a recombinant adeno-associated virus. As used herein, the term "adeno-associated virus" (AVV) includes any serotype of AAV. In general, serotypes of AAV have genomic sequences with a significant homology at the level of amino acids and nucleic acids, provide an identical series of genetic functions, produce virions that are essentially equivalent in physical and functional terms, and replicate and assemble through practically identical mechanisms. In particular, the invention may be performed using serotype 1 of AAV (AAV1), AAV2, AAV3 (including types 3A and 3B), AAV4, AAV5, AAV6, AAV7, AAV8, AAV9, AAV 10, AAV11, avian AAV, bovine AAV, canine AAV, equine AAV, ovine AAV, and any other AAV currently known or which may be discovered in the future. See, for example, Fields et al., Virology, volume 2, chapter 69 (4th ed., Lippincott-Raven Publishers). Recently, a number of putative new serotypes cloned from AAV have been identified (see, for example, Gao et al., (2004) J. Virology 78: 6381-6388; Moris et al., (2004) Virology 33: 375-383). The genomic sequences of several serotypes of AAV and autonomous parvoviruses, as well as the sequences of the inverted terminal repeats (ITR), Rep proteins and capsid sub-units are known in the state of the art. Such sequences may be found in the literature or in public databases such as GenBank. See, for example, GenBank Access Numbers NC_002077, NC_001401, NC_001729, NC_001863, NC_001829, NC_001862, NC_000883, NC_001701, NC_001510, NC_006152, NC_006261, AF063497, U89790, AF043303, AF028705, AF028704, J02275, J01901, J02275, X01457, AF288061, AH009962, AY028226, AY028223, NC_001358, NC_001540, AF513851, AF513852, AY530579; the information about them is incorporated herein by reference thereto for the teaching of nucleic acid and amino acid sequences from parvoviruses and AAV. See also, for example, Srivistava et al. (1983), J. Virology 45: 555; Chiorini et al. (1998). J. Virology 71: 6823; Chiorini et al. (1999), J. Virology 73: 1309; Bantel-Schaal et al. (1999), J. Virology 73: 939; Xiao et al. (1999), J. Virology 73: 3994; Muramatsu et al. (1996), Virology 221: 208; Shade et al. (1986), J. Virol. 58: 921; Gao et al. (2002). Proc. Nat. Acad. Sci. USA 99: 11854; Moris et al. (2004), Virology 33: 375-383; international patent publications WO 00/28061, WO 99/61601, WO 98/11244; and U.S. patent No. 6156303; the information about them is incorporated herein by reference thereto for a description of the nucleic acid and amino acid sequences of AAV.

Typically, the "recombinant AAV genome" (or "rAAV genome") refers to a vector that comprises one or more sequences of polynucleotides of interest, genes of interest or "transgenes", which are flanked by at least one inverted terminal repeat sequence (ITR) from parvovirus or AAV. Such rAAV vectors may replicate and package in infectious viral particles when they are present in a host cell that expresses the products of the rep and cap genes of AAV (that is, the Rep and Cap proteins of AAV). When an rAAV vector is incorporated into a large nucleic acid construct (for example, a chromosome or another vector, such as a plasmid or baculovirus used for cloning or transfection), the rAAV vector is typically referred to as a "pro-vector" that may be "rescued" by replication and encapsidation in the presence of the packaging functions of AAV and the necessary helper functions.

Thus, in a preferred form of embodiment, the recombinant viral genome of the invention comprises the gene construct of the invention and at least one ITR from AAV. Preferably, the gene construct of the invention is flanked by ITRs from AAV. The inverted terminal repeats (ITRs) are typically present in at least two copies in the AAV vector, typically flanking the gene construct of the invention. Typically, the ITRs will be located at the 5'- and 3'-ends of the gene construct of the invention, but need not be adjacent thereto. The terminal repeats may be identical or different from one another. The term "terminal repeat" includes any viral terminal repeat and/or partially or fully synthetic sequences that form hairpin structures and act as inverted terminal repeats, such as the "double D sequence" described in United States patent No. 5478745 to Salmulski et al. A "terminal repeat of AAV" may be derived from any AAV, including, but not limited thereto, serotypes 1, 2, 3, 4, 5, 6, 7, 8, 9, 10, 11, or 12, or any other AAV currently known or which may be discovered in the future. The terminal repeat of AAV neet not be a wild sequence (for example, a wild sequence may be altered by insertion, deletion, truncation or nonsensical mutations), whilst the terminal repeat mediates the desired functions, for example, replication, splicing, packaging of viruses, integration and/or rescue of pro-viruses, and similar functions. The vector genome may comprise one or more (for example, two) terminal repeats of AAV, which may be identical or different from one another. Moreover, one or more terminal repeats of AAV may be of the same AAV serotype as the AAV capsid, or may be different. In particular forms of embodiment, the vector genome comprises a terminal repeat of AAV1, AAV2, AAV3, AAV4, AAV5, AAV6, AAV7, AAV8, AAV9, AAV10, AAV11 and/or AAV12; in particular, AAV1, AAV2 and/or AAV4. In a preferred form of embodiment, the ITRs may be derived from AAV2 and may be defined by SEQ ID NO: 9 (5'-ITR) and SEQ ID NO: 10 (3'-ITR).

Although it is preferable that the nucleic acid sequences which encode the capsid genes are provided in *trans* by the packaging cell or by a second vector, the invention also considers using AAV genomes that additionally comprise a sequence that encodes one or more proteins of the capsid that packages the polynucleotide sequence mentioned above. The sequences that encode the VP1, VP2 and VP3 capsid proteins to be used in the context of this invention may come from any of the 42 serotypes known, more preferably from AAV1, AAV2, AAV3, AAV4, AAV5, AAV6, AAV7, AAV8 or AAV9, or newly developed particles similar to AAV obtained, for example, using capsid mixture techniques and AAV capsid libraries. When the sequences that encode the capsid proteins and the ITRs are derived from different AAV serotypes, the AAV genome is known as a "hybrid" AAV genome (that is, one wherein the AAV capsid and the terminal repeats of AAV are from different AAVs), as described in international patent publication WO 00/28004 and in Chao et al. (Molecular Therapy 2000, 2: 619). As described herein, the rAAV vector may be any adequate rAAV vector currently known or which may be discovered in the future. Alternatively, the sequences that encode the capsid genes may be provided in *trans* by co-transfection in the packaging cell of a polynucleotide that encodes said capsid proteins. In a preferred form of embodiment, the viral vector comprises ITRs from AAV1, AAV2 and/or AAV4, and one or more or all the capsid genes from AAV1, AAV2, AAV5, AAV6 or AAV8.

Optionally, the AAV genomes of the invention may comprise additional sequences that encode Rep proteins. The sequences that encode Rep (Rep78/68 and Rep52/40) are preferably derived from AAV1, AAV2 and/or AAV4. The Rep and ITR sequences of AAV are particularly preserved within most serotypes. The Rep78 proteins of several AAV serotypes are, for example, over 89% identical and the total identity of the nucleotide sequence between AAV2, AAV3A, AAV3B and AAV6 at the genome level is about 82% (Bantel-Schaal et al., 1999, J. Virol., 73: 939-947). Moreover, it is well known that the Rep sequences and the ITRs of many AAV serotypes effectively trans-complement (that is, functionally substitute for one another) the corresponding sequences of other serotypes in the production of AAV particles in mammalian cells. US2003148506 discloses that the Rep sequences and the ITRs of AAV also effectively trans-complement other Rep sequences and ITRs of AAV in insect cells.

It is well known that the VP proteins of AAV determine the cellular tropism of the AAV virion. The sequences that encode the VP proteins are significantly less preserved than the Rep proteins and genes amongst the different serotypes of AAV. The capacity of the Rep sequences and the ITRs to trans-complement the corresponding sequences of other serotypes allows for the production of pseudotyped rAAV particles which comprise the capsid proteins of one serotype (for example, AAV5) and the Rep and/or ITR sequences of another serotype of AAV (for example, AAV2). Such pseudotyped rAAV particles are a part of this invention.

Another aspect of the invention relates to a virion that may obtained by expressing a viral genome in accordance with this invention in an adequate packaging cell. The terms "virion", "recombinant virus particle" and "viral vector" are used interchangeably herein and refer to an infectious virus particle, deficient in replication, which comprises the viral genome packaged in a capsid and, optionally, in a lipid envelope surrounding the capsid. In a preferred form of embodiment, the virion is an AAV virion. In another form of embodiment, if the virion is obtained by packaging an AAV vector of the invention, the virion of the invention is a "recombinant AAV virion". The term "recombinant AAV virion" or "rAAV virion", as used herein, refers to an infectious virus, deficient in replication, composed of an AAV protein skeleton that encapsidates a polynucleotide which comprises the gene construct of the invention flanked on both ends by the ITRs of AAV.

The term "Cap protein", as used herein, refers to a polypeptide that has at least one functional activity of a native Cap protein from AAV (for example, VP1, VP2, VP3). Examples of the functional activities of Cap proteins (for example, VP1, VP2, VP3) include the capacity to induce the formation of a capsid, facilitate the accumulation of monocatenary DNA, facilitate the packaging of DNA from AAV in capsids (that is, encapsidation), bind to cellular receptors and facilitate the entry of the virion into the host cells. In a preferred form of embodiment, the polynucleotide sequence that encodes the cap gene corresponds to the cap gene of AAV8. The skeleton of an AAV virion exhibits icosahedral symmetry and normally contains a main Cap protein, normally the smaller Cap protein, and one or two minority Cap proteins.

The term "Rep protein", as used herein, refers to a polypeptide that has at least one functional activity of a native Rep protein from AAV (for example, Rep 40, 52, 68, 78). A "functional activity" of a Rep protein (for example, Rep 40, 52, 68, 78) is any activity associated with the protein's physiological function, including facilitating DNA replication by recognition, binding and splicing of the DNA replication origin from AAV, as well as DNA helicase activity. Additional functions include the modulation of the transcription of AAV promoters (or other heterologues) and site-specific AAV DNA integraton into a host chromosome. In a preferred form of embodiment, the polynucleotide sequence that encodes the rep gene corresponds to the rep gene from AAV2.

Those skilled in the art will understand that the AAV virions of the invention may comprise capsid proteins from any serotype of AAV. However, due to the different tropism of the known serotypes of AAV for different cells, the AAV virions will contain a capsid protein that is more adequate for distribution to the hepatic cells. For the transduction of hepatic cells, rAAV virions with capsid proteins from AAV1, AAV8 and AAV5 are preferred (Nathwani et al., 2007, Blood 109: 1414-1421; Kitajima et al., 2006, Atherosclerosis 186: 65-73).

The sequences that encode Rep (Rep78/68 and Rep52/40) may be from any serotype of AAV, but are preferably derived from AAV1, AAV2 and/or AAV4. However, the sequences that encode the VP1, VP2 and VP3 capsid proteins to be used in the context of this invention may be obtained from any of the 42 known serotypes, more preferably from AAV1, AAV2, AAV5, AAV6 or AAV8.

The invention also considers virions that comprise a capsid and a recombinant viral genome, wherein an exogenous targeting sequence has been inserted or substituted in the native capsid. The virion is preferably targeted (that is, targeted to a particular type or types of cells) by means of the substitution or insertion of the exogenous targeting sequence in the capsid. In other words, the exogenous targeting sequence preferably confers an altered tropism to the virion. As an additional alternative explanation, the targeting sequence increases the distribution efficiency of the vector targeted to a cell.

The exogenous targeting sequence(s) may change or substitute all or part of a capsid subunit; alternatively, more than one capsid subunit. As an additional alternative, more than one exogenous targeting sequence (for example, two, three, four, five or more sequences) may be introduced into the virion capsid. In alternative forms of embodiment, insertions and substitutions in the minority capsid subunits are preferred (for example, VP1 and VP2 ofAAV). For AAV capsids, insertions or substitutions in VP2 and VP3 are also preferred.

In more preferred forms of embodiment, the exogenous targeting sequence may be an amino acid sequence that encodes a peptide or protein, which is inserted or substituted in the virion capsid in order to change the tropism of the virion. The tropism of the native virion may be reduced or eliminated by the insertion or substitution of the amino acid sequence. Alternatively, the insertion or substitution of the exogenous amino acid sequence may target the virion to a particular type of cells. The exogenous targeting sequence may be any amino acid sequence that encodes a protein or peptide that changes the tropism of the virion. In particular forms of embodiment, the targeting peptide or protein may be of natural origin or, alternatively, fully or partially synthetic. Exemplary peptides and proteins include ligands and other peptides that bind to cell surface receptors present in liver cells, including ligands capable of binding to the Sr-B1 receptor for apolipoprotein E, galactose and lactose-specific lectins, ligands from the low-density lipoprotein receptor, ligands from asialoglycoprotein (terminal galactose) and similar ones.

Alternatively, the exogenous targeting sequence may be an antibody or a group antigen recognition thereof. The term "antibody", as used herein, refers to all types of immunoglobulins, including IgG, IgM, IgA, IgD and IgE. The antibodies may be monoclonal or polyclonal, and may be from any species of origin, including (for example) mouse, rat, rabbit, horse or human being, or may be chimeric antibodies. The term "antibody" also includes bi-specific or "bridge" antibodies known to those skilled in the art. The antibody fragments within the scope of this invention include, for example, fragments Fab, F(ab')2 and Fc, and the corresponding fragments obtained from different IgG antibodies. Such fragments may be produced by techniques known in the state of art. Hepatic surface markers that may be used for the targeting of the rAAVs of the invention include, without limitation, the hepatitis B virus and the LDL surface antigen.

The exogenous amino acid sequence inserted in the virion capsid may be one that facilitates the purification or detection of the virion. In accordance with this aspect of the invention, it is not necessary for the exogenous amino acid sequence to also change the modified parvovirus virion. For example, the exogenous amino acid sequence may include a polyhistidine sequence that is useful to purify the virion on a nickel column, as known to those skilled in the art, or an antigenic peptide or protein may be used to purify the virion by standard immunopurification techniques. Alternatively, the amino acid sequence may encode a receptor ligand or any other peptide or protein that may be used to purify the modified virion by affinity purification or any other method known in the state of the art (for example, purification techniques based on size, density, charge, or differential isoelectric point, ion-exchange chromatography, or peptide chromatography). The insertions of exogenous targeting or purification sequences may be performed in any capsid protein, provided that the insertion does not involve said protein's capacity to assemble. In particular, it is preferable to insert the exogenous amino acid sequence in the minority Cap subunits of AAV, for example, in the VP1 and VP2 subunits of AAV. Alternatively, it is possible to perform the insertions in VP2 or VP3. The preferred AAV virions may be modified to reduce the host response (see, for example, Russell (2000, J. Gen. Virol. 81: 2573-2604), US20080008690, and Zaldumbide and Hoeben (Gene Therapy, 2008: 239-246)).

The recombinant virions of the invention may be prepared using standard technology for the preparation of AAVs. Typically, the rAAVs are prepared by the introduction of the viral genome in accordance with the invention into an adequate host cell and the co-expression, in said cell, of a rep protein of AAV, a cap protein of AAV and, optionally, a nucleic acid sequence that encodes viral and/or cellular functions whereon AAV is dependent for replication.

In order to facilitate the packaging, the recombinant vector genome is generally between about 80% and 105% of the wild genome size and comprises an adequate packaging signal. In order to facilitate packaging in an AAV capsid, the genome is preferably approximately 5.2 kb in size or less. In other forms of embodiment, the genome is preferably greater than about 3.6, 3.8, 4.0, 4.2 or 4.4 kb in length and/or less than about 5.4, 5.2, 5.0 or 4.8 kb in length. In other words, the heterologous nucleotide sequence(s) will be typically less than 5.0 kb in length (more preferably, less than about 4.8 kb, even more preferably, less than about 4.4 kb in length, even more preferably less than about 4.2 kb in length) in order to facilitate packaging of the recombinant genome by the AAV capsid.

The nucleic acid sequences necessary for the production of the virion of the invention are the so-called "AAV helper functions" and comprise one or both of the main ORFs of AAV, that is, the regions that encode rep and cap, or functional homologues thereof. Adequate nucleic acid sequences that encode the rep and cap proteins to be used in the method of the invention have been described in detail above, in relation to the virions of the invention. Those skilled in the art will note, however, that the helper sequences that encode the rep and cap proteins of AAV may be provided by one, two or more vectors in several combinations. As used herein, the term "vector" includes any genetic element, such as a plasmid, phage, transposon, cosmid, chromosome, artificial chromosome, virus, virion, etc., that is capable of replication when associated with the adequate control elements and which may transfer gene sequences between cells. Thus, the term includes cloning and expression vehicles, as well as viral vectors. Alternatively, the rep and/or cap genes of AAV may be provided by a packaging cell that expresses these genes in a stable manner (see, for example, Gao et al. (1998), Human Gene Therapy 9: 2353; Inoue et al. (1998), J. Virol. 72: 7024; U.S. patent No. 5837484; WO 98/27207; U.S. patent No. 5658785; WO 96/17947).

In a preferred form of embodiment, the polynucleotides that encode the rep and cap proteins may be provided by a single individual vector, which is normally referred to as an AAV helper function vector. Examples of vectors suitable to be used with this invention include pHLP19, described in U.S. patent No. 6001650, and the pRep6cap6 vector, described in U.S. patent No. 6156303, the complete information whereof is incorporated herein by means of a reference.

In other particular forms of embodiment, the additional sequences are in the form of a helper adenovirus virus which may be a hybrid helper virus that encodes the Rep and/or capsid proteins of AAV. The Ad/AAV hybrid helper vectors that express the rep and/or cap genes of AAV and methods to produce AAV reserves using these reagents are known in the state of the art (see, for example, U.S. patent No. 5589377; and U.S. patent No. 5871982, U.S. patent No. 6251677; and U.S. patent No. 6387368). Preferably, the hybrid Ad of the invention expresses the capsid proteins of AAV (that is, VP1, VP2 and VP3). Alternatively, or additionally, the hybrid adenovirus may express one or more Rep proteins of AAV (that is, Rep40, Rep52, Rep68 and/or Rep78). The AAV sequences may be operatively associated with a tissue-specific or inducible promoter.

The optional component for the generation ofrecombinant virions may comprise a nucleic acid sequence that encodes viral functions not derived from AAV and/or cellular functions whereon AAV depends for replication (that is, "accessory functions"). Accessory functions include those functions required for the replication of AAV, including, without limitation, those groups involved in the activation of AAV gene transcription, phase-specific adjustment of AAV mRNA, replication of AAV DNA, synthesis of cap expression products and assembly of the AAV capsid. The virus-based accessory functions may be derived from any of the known helper viruses, such as adenoviruses, herpes viruses (different from the herpes simplex virus type 1) and vaccine viruses. Typically, the AAV vector packaging plasmid in accordance with the invention contains, as helper virus DNA sequences, the E2A, E4 and VA genes of Ad5, which may be derived from the pDG plasmid disclosed in German patent application DE196 44 500.0-41, and which are controlled by the respective original promoter or by heterologous promoters.

Alternatively, it is possible to express the structural components of AAV and put these in contact with the viral genome of the invention in such a way that encapsidation takes place *in vitro.* In order to prepare structural proteins, any type of suitable host cell may be used. Preferably, insect cells are used, as described by Urabe et al. (Hum. Gene Ther. 2002, 13: 1935-1943; US6723551 and US20040197895). Cell lines suitable for the expression of the structural components of rAAV include, without limitation, *Spodoptera frugiperda* cell lines, Drosophila cell lines or mosquito cell lines, for example, cell lines derived from *Aedes albopictus.* The preferred insect cells or cell lines are from insect species that are susceptible to infection by baculoviruses, including, for example, Se301, SeIZD2109, SeUCR1, Sf9, Sf900+, Sf21, BTI-TN-5B1-4, MG-I, Tn368, HzAmI, Ha2302, Hz2E5, High Five (Invitrogen, CA, USA) and expresSF+® (US 6.103.526; Protein Sciences Corp., CT, USA).

Once the virions of the invention have been assembled, it is possible to purify them in order to separate them from those components that have not become a part of the virions. Typically, the virions are separated from the rest of the components by means of a density gradient, typically an iodixanol gradient. Following the recovery of the fraction that contains the rAAVs, the subsequent purification thereof is possible using chromatography, which may be ion-exchange or hydroxyapatite chromatography. This type of purification is that preferred for the purification of virions wtih capsids that contain proteins of serotypes 1 and 5 of AAV, because these serotypes do not bind to heparin columns.

For the purification of rAAV2 virions, heparin-agarose chromatography is preferred. See, for example, U.S. patent No. 6146874.

The virions are also purified using chromatography in the absence of density gradient centrifugation. As an example, the lysates of infected cells may be directly subjected to chromatography for the purification of rAAV virions. For methods of large-scale production of rAAV vectors that involve chromatography, see Potter et al. (Methods Enzymol., 2002, 346: 413-430).

The recombinant virions may be used or the virion vectors may be subjected to an additional affinity purification step, using an anti-AAV antibody, preferably an immobilised antibody. The anti-AAV antibody is preferably a monoclonal antibody. A particularly suitable antibody is a camelid single-chain antibody or a fragment thereof, which may be obtained, for example, from camels or llamas (see, for example, Muyldermans, 2001, Biotechnol. 74: 277-302). Preferably, the antibody for the affinity purification of rAAV is an antibody that specifically binds to an epitope of a capsid protein of AAV, wherein the epitope is, preferably, an epitope that is present in capsid proteins of more than one serotype of AAV. For example, the antibody may be produced or selected on the basis of specific binding to the AAV2 capsid, but, at the same time, it may also specifically bind to the capsids of AAV1, AAV3 and AAV5.

### *IN VITRO* METHOD FOR THE *IN VITRO* EXPRESSION OF A POLYNUCLEOTIDE OF INTEREST

The gene constructs, vectors and virions of the invention allow for the *in vitro* expression of polynucleotides of interest in a cell of hepatic origin. Therefore, another aspect of the invention relates to an *in vitro* method for the expression of a polynucleotide of interest in a cell of hepatic origin, which comprises the following steps:

(i) placing said cell in contact with a gene construct of the invention, a vector of the invention, a viral genome of the invention or a virion of the invention under adequate conditions for said construct, said vector or said virion to enter into the cell, and
(ii) putting the cell in contact with the inducer agent for the necessary time for the expression of the polynucleotide of interest to take place.

Cells of hepatic origin wherein a polynucleotide of interest may be expressed using the *in vitro* method of this invention include not only cells from primary hepatocyte cultures, but also immortalised cells of hepatic origin, such as cell lines from HepG2 hepatoma, COLO 587, FaO, HTC, HuH-6, HuH-7, PLC, Hep3B, BPRCL, MCA-RH777, BEL-7404, SMMC-7221, L-02, CYNK-1, PLC/PRF/5 and MCA-RH8994, as well as lines experimentally immortalised by the expression of viral or cellular oncogenes, such as cells from the Fa2N-4 and Ea1C-35 lines.
The method of *in vitro* expression in accordance with the invention comprises a first step wherein the cell of hepatic origin is placed in contact with a gene construct, a vector, a viral genome or a virion of the invention under adequate conditions for the entry of said construct, said vector or said virion into the cell. Suitable methods to promote the entry of a nucleic acid into the interior of a cell include, without limitation, the direct injection of naked DNA, ballistic methods, liposome-mediated transfer, receptor-mediated transfer (ligand-DNA complex), electroporation and precipitation with calcium phosphate (see, for example, US 4970154, WO 96/40958, US 5679559, US 5676954 and US 5593875). In the event that the gene construct is supplied in the form of a virion, the entry of the genetic material into the cell takes place thanks to the intrinsic capacity of virions to bind to the surface of the hepatic cell and release the genetic material in the interior thereof. In this regard, as mentioned above, it is preferable to use rAAVs whose capsid proteins belong to serotypes AAV1, AAV8 and AAV5, as previously described (Nathwani et al., 2007, Blood 109: 1414-1421; Kitajima et al., 2006, Atherosclerosis 186: 65-73).
In the second step of the method of the invention, the cells that contain the gene construct of the invention inside them are placed in contact with an inducer agent, such that the transcription of both the transactivator and the polynucleotide of interest is activated. The optimal concentration of inducer agent, as well as the adequate incubation time of the cells with said inducer agent must be experimentally determined.
The expression of the polynucleotide of interest in response to the inducer agent may be determined using techniques known to those skilled in the art for the determination of mRNA levels in a sample (RT-PCR, Northern blot and similar techniques) or for the determination of protein levels (ELISA, Western blot, RIA and similar techniques).

PHARMACEUTICAL COMPOSITIONS AND THERAPEUTIC USES OF THE INVENTION

The compounds of the invention are useful for the temporally-controlled hepato-specific expression of products with therapeutic interest. Therefore, another aspect of the invention relates to a pharmaceutical preparation that comprises a therapeutically effective quantity of a gene construct of the invention, a vector of the invention, a virion of the invention and a pharmaceutically acceptable vehicle (carrier) or excipient.
Another aspect of the invention relates to a gene construct of the invention, a vector of the invention or a virion of the invention to be used in medicine.
The pharmaceutical compositions of the invention may be administered by any route, including, but not limited thereto, oral, intravenous, intramuscular, intra-arterial, intramedullar, intrathecal, intraventricular, transdermal, subcutaneous, intraperitoneal, intranasal, enteric, topical, sublingual or rectal route. A review of the different forms of administration of active principles, the excipients to be used and the methods of manufacturing them may be found in "Tratado de Farmacia Galénica", C. Faulí i Trillo, Luzán 5, S.A. de Ediciones, 1993, and in "Remington's Pharmaceutical Sciences" (A.R. Gennaro, Ed.), 20th edition, Williams & Wilkins PA, USA (2000). There are examples of pharmaceutically acceptable carriers known in the state of the art, which include phosphate-buffered saline solutions, water, emulsions, such as oil/water emulsions, different types of wetting agents, sterile solutions, etc. The compositions that comprise said carriers may be formulated by conventional methods known in the state of the art.
In the event that nucleic acids (the polynucleotides, the vectors, the gene constructs or the viral vectors of the invention) are administered, the invention considers pharmaceutical compositions especially prepared for the administration of said nucleic acids. The pharmaceutical compositions may comprise said nucleic acids in naked form, that is, in the absence of compounds that protect the nucleic acids from degradation by the body's nucleases, which has the advantage that the toxicity associated with the reagents used for the transfection is eliminated. Suitable administration routes for the naked compounds include intravascular, intratumoural, intracraneal, intraperitoneal, intrasplenic, intramuscular, subretinal, subcutaneous, mucosal, topical and oral (Templeton, 2002, DNA Cell Biol., 21: 857-867). Alternatively, the nucleic acids may be administered as a part of liposomes, conjugated with cholesterol or conjugated with compounds capable of promoting translocation through cell membranes, such as the Tat peptide derived from the TAT protein of HIV-1, the third helix of the homeodomain of the Antennapedia protein of D. melanogaster, the VP22 protein of the herpes simplex virus, oligomers of arginine and peptides such as those described in WO07069090 (Lindgren, A.

et al., 2000, Trends Pharmacol. Sci, 21: 99-103; Schwarze, S.R. et al., 2000, Trends Pharmacol. Sci., 21: 45-48; Lundberg, M. et al., 2003, Mol. Therapy 8: 143-150; and Snyder, E.L. and Dowdy, S.F., 2004, Pharm. Res. 21: 389-393).

In the event that virions are administered, the quantity and the administration time thereof will depend on the circumstances and must be optimised in each case by the person skilled in the art using standard technology. Thus, it is possible to administer therapeutically effective quantities of virions of the invention by a single administration, such as, for example, a single injection of a sufficient number of infectious particles in order to provide therapeutic benefit to the patient subject to such treatment. Alternatively, in some circumstances, it may be desireable to supply multiple or successive administrations of the virion compositions, either for a relatively short or a relatively prolonged period of time, as may be determined by the physician supervising the administration of such compositions. For example, the number of infectious particles administered to a mammal may be of the order of about $10^7$, $10^8$, $10^9$, $10^{10}$, $10^{11}$, $10^{12}$, $10^{13}$, or even more, infectious particles/ml supplied in a single dose, or divided into two or more administrations, as may be required for the therapy of the particular disease or disorder that is to be treated. In fact, in certain forms of embodiment, it may be desireable to administer two or more compositions of different virion vectors, either by themselves or in combination with one or more drugs, in order to achieve the desired effects of the particular therapeutic pattern. In most virion-based gene therapy patterns, the use of a liver-specific promoter to control the expression of the polynucleotide of interest will result in a lower title of infectious particles being required when the virions in accordance with the invention are used as compared to conventional gene therapy protocols.

In another form of embodiment, the compositions and polynucleotides of the invention are administered by the so-called "hydrodynamic administration", as it has been described by Liu, F., et al. (Gene Ther, 1999, 6: 1258-66). According to said method, the compounds are introduced into the body at high speed and volume by intravascular route, which leads to high transfection levels with a more diffuse distribution. It has been proven that the efficacy of intracellular access is directly dependent on the volume of fluid administered and on the injection speed (Liu et al., 1999, Science, 305: 1437-1441). In mice, the administration has been optimised at values of 1 ml/10 g of body weight in a period of 3-5 seconds (Hodges et al., 2003, Exp. Opin. Biol. Ther, 3: 91-918). The exact mechanism that allows for in *vivo* cell transfection with polynucleotides following the hydrodynamic administration thereof is as yet not completely known. In the case of mice, it is believed that administration through the tail vein takes place at a rate that exceeds the heart rate, which causes the administered fluid to accumulate in the superior vena cava. This fluid subsequently accesses the organ vessels and, subsequently, through fenestrations in said vessels, accesses the extravascular space. In this way, the polynucleotide comes in contact with the target organ cells prior to mixing with the blood, thereby reducing the possibility of degradation by nucleases.

The compositions of the invention may be administered in doses of less than 10 mg per kilogram of body weight, preferably less than 5, 2, 1, 0.5, 0.1, 0.05, 0.01, 0.005, 0.001, 0.0005, 0.0001, 0.00005 or 0.00001 mg for every kg of body weight, and less than 200 nmol of RNA agent, that is, about 4.4 x $10^{16}$ copies per kg of body weight or less than 1,500, 750, 300, 150, 75, 15, 7.5, 1.5, 0.75, 0.15 or 0.075 nmol per kg of body weight. The unit doses may be administered by injection, by inhalation or by topical administration. In the event that AAV virions are administered, these may be systemically administered, since, thanks to their tropism for hepatic cells, they will access this organ. However, in the event that the gene constructs of the invention or the plasmids of the invention are administered, these must be preferably administered to the liver in a targeted manner by administration in the hepatic artery or other hepatic administration systems known in the state of the art, such as those described by Wen et al. (World J. Gastroenterol, 2004, 10, 244-9), Murao et al. (Pharm. Res., 2002, 19, 1808-14), Lin et al. (Gene Ther., 2003, 10, 180-7), Hong et al. (J. Pharm. Pharmacol., 2003, 54, 51-8), Herrmann et al. (Arch Virol, 2004, 149, 1611-7) and Matsuno et al. (Gene Ther, 2003, 10, 1559-66). The compositions may be administered at doses of between 0.00001 mg and 3 mg, preferably between 0.0001 and 0.001 mg, even more preferably of about between 0.03 and 3.0 mg per organ, of about between 0.1 and 3.0 mg per organ or between 0.3 and 3.0 mg per organ.

The dose of the compositions of the invention to be administered depends on the severity and response of the condition to be treated and may vary between several days and several months, or until it is observed that the condition subsides. The optimal dosage may be determined by periodically measuring the agent concentrations in the patient's body. The optimal dose may be determined from the EC50 values obtained by means of previous in *vitro* or in *vivo* assays in animal models. The unit dose may be administered once a day or less than once a day, preferably, less than once every 2, 4, 8 or 30 days. Alternatively, it is possible to administer an initial dose followed by one or several maintenance doses, generally in a lower quantity than the initial dose. The maintenance scheme may involve treating the patient with doses that range between 0.01 $\mu$g and 1.4 mg/kg of body weight per day, for example, 10, 1, 0.1, 0.01, 0.001 or 0.00001 mg per kg of body weight per day. The maintenance doses are preferably administered at most once every 5, 10 or 30 days. The treatment must be continued for a period of time that will vary depending on the type of alteration that the patient suffers, the severity thereof and the patient's condition. Following the treatment, the patient's evolution must be monitored in order to determine whether the dose must be increased, in the event that the disease does not respond to the treatment, or the dose is reduced, if an improvement of the disease is observed or undesireable secondary effects are observed.

The daily dose may be administered in a single dose or in two or more doses, depending on the particular circumstances. If repeated administration or frequent administrations are desired, the implantation of an administration device, such as a pump, a semi-permanent catheter (intravenous, intraperitoneal, intracisternal or intracapsular) or a reservoir, is adviseable.

Given that the constructs of the invention allow for the expression of a polynucleotide of interest in a regulated manner when the cell that contains said construct is placed in contact with an inducer agent, the therapeutic uses of the gene constructs of the invention consider a second step for the administration of the inducer agent. The inducer agent may be administered in the form of a prodrug, salt, solvate or clathrate, either in isolated form or in combination with additional active agents. The preferred excipients to be used in this invention include sugars, starches, celluloses, rubbers and proteins. The inducer agents may be administered formulated in a solid administration pharmaceutical form (for example, tablets, capsules, pills, granules, suppositories, crystalline or amorphous sterile solids that may be reconstituted to provide liquid forms, etc.), a liquid administration pharmaceutical form (for example, solutions, suspensions, emulsions, elixirs, lotions, ointment, etc.) or a semi-solid administration pharmaceutical form (gels, ointments, creams and similar forms). The dose of inducer agent, the administration route and the waiting time between the administration of the gene construct or the virions and the administration of the inducer agent may be routinely determined in each specific case by the person skilled in the art.

Given the capacity of the constructs of the invention to allow for temporally or spatially regulated expression in the liver, these constructs are particularly suitable for the expression in the liver of polypeptides the function whereof is useful for the treatment and prevention of hepatic diseases. Thus, another aspect of the invention relates to a gene construct of the invention, a vector of the invention, a viral genome of the invention, a virion of the invention or a pharmaceutical composition of the invention to be used in the treatment of a hepatic disease. Alternatively, the invention relates to the use of a gene construct of the invention, a vector of the invention, a viral genome of the invention, a virion of the invention or a pharmaceutical composition of the invention in the manufacturing of a drug to be used in the treatment of a hepatic disease. Alternatively, the invention relates to a method designed for the treatment of a hepatic disease which comprises the administration of a gene construct of the invention, a vector of the invention, a viral genome of the invention, a virion of the invention or a pharmaceutical composition of the invention to a subject who needs it.

The term "treatment", as used herein, refers to the act of reversing, improving or inhibiting the evolution of the disorder or condition whereto such term is applied, or of one or more symptoms of such disorder or condition.

The term "prevention", as used herein, refers to the act of preventing the occurrence or existence, or, alternatively, of delaying the beginning or reappearance of a disease, disorder or condition whereto said term is applied, or of one or more symptoms associated with a disease, disorder or condition.

The hepatic disorders that may be adequately treated or prevented by using the constructs, vectors and virions of the invention are shown in Table 1, jointly with the polypeptide that should be encoded by the polynucleotide of interest:

Table 1: Polynucleotides of interest that may be incoporated into the gene constructs of the invention and disorders wherein they may be used.

| Polypeptide encoded by the polynucleotide of interest | Disease |
|---|---|
| *IFNa5* | chronic hepatitis C<br>chronic hepatitis B<br>vaccine adjuvant<br>hepatocarcinoma |
| *Oncostatin M* | chronic hepatitis C<br>chronic hepatitis B<br>hepatocarcinoma |
| *Cardiotrophin* | Hepatic transplantation<br>Renal transplantation<br>Hepatectomies<br>chronic hepatitis C<br>chronic hepatitis B<br>hepatocarcinoma |
| *IL6* | Hepatic transplantation<br>Renal transplantation<br>Hepatectomies |
| *Amphiregulin* | Hepatic transplantation |

(continued)

| Polypeptide encoded by the polynucleotide of interest | Disease |
|---|---|
| | Hepatectomies |
| *EDA:* | Vaccine adjuvant |
| *IL15* | Immunotherapy adjuvant<br>chronic hepatitis C<br>chronic hepatitis B<br>hepatocarcinoma |
| *IL12* | Hepatocarcinoma<br>Immunotherapy adjuvant<br>chronic hepatitis C<br>chronic hepatitis B |
| *CD134:* | Immunotherapy adjuvant |
| *CD137:* | Immunotherapy adjuvant |
| *PBGD:* | Acute intermittent porphyria |
| *p17(TGF-β1 inhibitor)* | Adjuvant in colon cancer<br>Pulmonary Fibrosis<br>Bone metastasis |
| *p144 rTGF-β1 inhibitor)* | Adjuvant in colon cancer<br>Mammary prostheses<br>Systemic sclerosis<br>Morphea<br>Burns<br>Cardiac fibrosis<br>Renal fibrosis |
| *IL10 inhibitors* | Viral infections<br>Bacterial infections<br>Parasitic infections<br>Non-Hodgkin's lymphoma |
| *FoxP3 inhibitors* | Immunotherapy adjuvant<br>(blocking regulatory T cells) |
| *TNFα inhibitors* | Rheumatoid arthritis |
| *VEGF inhibitors* | Anti-angiogenesis |
| *PD-1 inhibitors* | Immunotherapy adjuvant |
| *CD152 inhibitors* | Immunotherapy adjuvant |
| *IGF-I* | Cirrhosis |

In a preferred form of embodiment, the polynucleotide of interest encodes IL-12 or a functionally equivalent variant, in which case the gene construct of the invention, a vector of the invention, the viral genome of the invention, the virion of the invention or the pharmaceutical composition of the invention are used for the treatment of hepatic cancer. The term "hepatic cancer", as used in this invention, refers to both primary cancer and secondary cancer, including that formed from any type of primary tumour. The types of hepatic cancer include, without limitation, hepatocellular carcinoma (sometimes called hepatoma or HCC), carcinoma, fibrolamellar HCC, cholangiocarcinoma, hemangioma, hepatic adenoma, focal nodular hyperplasia, angiosarcoma and hepatoblastoma.

CONSTRUCTS AND VECTORS OF THE INVENTION FOR GENERAL USE

Those skilled in the art will note that the bi-directional hepato-specific promoter in accordance with the invention must not necessarily form a part of a gene construct that additionally comprises a transcription activator and a polynucleotide of interest, but may be used in isolation as an integral element of other vectors, viral genomes or gene constructs.

Thus, another aspect of the invention relates to an inducible bi-directional operator-promoter suitable for the inducible hepato-specific expression of two polynucleotides of interest by an inducer agent, which comprises

(i) at least one responsive element to the transactivator in its active form, that is, in the presence of the inducer,
(ii) a first hepato-specific promoter sequence and
(iii) a second hepato-specific promoter sequence,

wherein the first and the second hepato-specific promoter sequences act in a divergent manner with respect to the responsive element to the inducer agent and wherein the promoter activity of the first and the second hepato-specific promoter sequences increases in the presence of a transactivator which, following administration of the inducer agent, binds to the responsive element.

The elements that compose the inducible bi-directional operator, specifically, the responsive element to the transactivator in its active form, the first hepato-specific promoter sequence and the second hepato-specific promoter sequence have been described in detail above and are interpreted in the same manner as that described above in relation to the gene construct of the invention.

In a preferred form of embodiment, the regulatable bi-directional operator-promoter comprises at least one responsive element to transactivator + tetracycline. In an even more preferred form of embodiment, the tetracycline responsive element comprises a nucleic acid sequence defined in SEQ ID NO: 1.

In another preferred form of embodiment, the first hepato-specific promoter sequence and the second hepato-specific promoter sequence are identical. In an even more preferred form of embodiment, the first hepato-specific promoter sequence and the second hepato-specific promoter sequence comprise the albumin gene promoter or a functionally equivalent variant thereof. In an even more preferred form of embodiment, the albumin gene promoter comprises a sequence selected from the group formed by SEQ ID NO: 2 and SEQ ID NO: 3.

In another preferred form of embodiment, the inducible bi-directional operator-promoter comprises SEQ ID NO: 4.

Thus, another aspect of the invention relates to a gene construct suitable for the inducible hepato-specific expression of a polynucleotide of interest by an inducer agent, which comprises

(a) An inducible bi-directional operator-promoter that comprises

(i) at least one responsive element to the transactivator in its active form, that is, in the presence of the inducer,
(ii) a first hepato-specific promoter sequence and
(iii) a second hepato-specific promoter sequence,

(b) one nucleotide sequence that encodes a transactivator which may be activated by said inducer agent that is operatively coupled to the first hepato-specific promoter sequence and a polyadenylation signal located at the 3' position with respect to the region that encodes the transactivator,

wherein the first and the second hepato-specific promoter sequences act in a divergent manner with respect to the responsive element to the inducer agent and wherein the promoter activity of the first and the second hepato-specific promoter sequences increases in the presence of said inducer agent and in the presence of a transactivator that binds to the responsive element in the inducible bi-directional operator-promoter.

The elements that compose the inducible bi-directional operator, specifically, the responsive element to the inducer agent, the type of transactivator, the first hepato-specific promoter sequence, the second hepato-specific promoter sequence and the polyadenylation signal have been described in detail above and are interpreted in the same manner as that described above in relation to the first gene construct of the invention.

In a preferred form of embodiment, the regulatable bi-directional operator-promoter comprises at least one tetracycline-responsive element. In an even more preferred form of embodiment, the tetracycline-responsive element comprises a nucleic acid sequence defined in SEQ ID NO: 1.

In another preferred form of embodiment, the transactivator is a reverse tetracycline-dependent transactivator. In an even more preferred form of embodiment, the reverse transactivator rtTA which may be activated by tetracycline is rtTA-M2.

In another preferred form of embodiment, the first hepato-specific promoter sequence and the second hepato-specific promoter sequence are identical. In an even more preferred form of embodiment, the first hepato-specific promoter sequence and the second hepato-specific promoter sequence comprise the albumin gene promoter or a functionally equivalent variant thereof. In an even more preferred form of embodiment, the albumin gene promoter comprises a sequence selected from the group formed by SEQ ID NO: 2 and SEQ ID NO: 3.

In another preferred form of embodiment, the inducible bi-directional operator-promoter comprises SEQ ID NO: 4.

In another preferred form of embodiment, the polyadenylation signal is a bi-directional polyadenylation signal. In an even more preferred form of embodiment, the polyadenylation signal is a bi-directional polyadenylation signal from the SV40 virus.

The second gene construct of the invention may be supplied in isolated form or, preferably, may be supplied as a part of a vector, in order to facilitate the propagation and manipulation thereof. In a preferred form of embodiment, the vector additionally comprises, at the 3' position with respect to the second hepato-specific promoter sequence, one or several sites that allow for the cloning of polynucleotides of interest such that they may be expressed in a hepato-specific manner in response to the addition of the activator agent. Preferably, the cloning sites are grouped so as to form a multiple cloning site, as they frequently appear in cloning vectors. Thus, the term "multiple cloning site", as used in this invention, refers to a nucleic acid sequence that comprises a series of two or more restriction endonuclease target sequences that are located close to one another. Multiple cloning sites include restriction endonuclease targets which allow for the insertion of fragments with blunt ends, sticky 5'-ends or sticky 3'-ends. The insertion of polynucleotides of interest is performed using standard molecular biology methods, as described, for example, by Sambrook et al. *(supra).*

The invention is described below by means of the following examples, which have a merely illustrative character and are in no case intended to limit the invention.

## EXAMPLES

### Example 1. Construction and characterisation of the recombinant rAAV-pTet$_{bidi}$-pCMV-luc virus.

The main objective was to obtain an inducible vector based on AAV8 which would make it possible to regulate the transgene expression in time, by varying the dose of inducer administered, and which would specifically target the transgene expression to hepatocytes, thereby acting at the target site for our therapy and preventing potentially toxic adverse effects of the transgene. Given that AAV8 primarily transduces the liver with a great efficiency, we set out to determine whether the rAAV-pTet$_{bidi}$-pCMV-luc system met the characteristics explained above when administered by intravenous route through the tail vein.

This system had been previously characterised by Chtarto *et al.* (Chtarto, A., et al. Gene Ther, 2003. 10: 84-94), but it had been administered intracerebrally, for which reason the biodistribution of the system following the systemic administration thereof was unknown.

To this end, the luciferase gene was used as the reporter gene, which made it possible to analyse the biodistribution of the transgene expression *in vivo,* in addition to allowing for the quantification thereof. To this end, the eGFP gene present in the plasmid, gently supplied by Dr. Lilianne Tenenbaum at the Frree University of Brussels, was replaced with the firefly luciferase gene, in order to obtain the rAAV-pTet$_{bidi}$-pCMV-luc system (Figure 2.A), wherewith we produced the rAAV2/8-pTet$_{bidi}$-pCMV-luc virions.

### 1.1 Construction of the vector

In detail, the plasmid that contains the recombinant AAV genome with the inducible pTet$_{bidi}$-pCMV-luc (pAC1M2-pCMV-luc) system was generated in the following manner: the luciferase gene was amplified from the pA1b-luc plasmid (Kramer G. et al. Molecular Therapy 2003, 7: 375-385) with primers A (sense primer) and B (anti-sense primer) (A: GTCGAC ATG GAA GAC GCC AAA AAC (SEQ ID NO: 11) and B: GCGGCCGC TTA CAC GGC GAT CTT TCC (SEQ ID NO: 12)), which, at the 5'-ends, contain the SalI (sense primer) and NotI (anti-sense primer) sites. This fragment was sub-cloned in the pCDNA3.1/V5-His TOPO TA cloning vector (Invitrogen), and was extracted therefrom by digestion with the enzymes mentioned above. The fragment extracted was inserted in the pAC1M2-EGFP vector (Chtarto, A., et al. Gene Ther, 2003. 10: 84-94; Chtarto, A., et al. Exp Neurol, 2007, 204: 387-399) which was previously digested with the same enzymes, to obtain the pAC1M2-pCMV-luc plasmid.

Once the plasmid containing the pTet$_{bidi}$-pCMV-luc system was obtained, rAAV2/8 virions were produced by transfection with PEI in HEK 293T cells, in accordance with the following protocol:

Twenty-four hours prior to the transfection, $8.5 \times 10^6$ cells/plate were plated in complete DMEM medium, in 150-mm-diameter plates (approximately 30 plates/production), in order to achieve a 70%-80% confluence at the time of transfection. Just before the transfection, the medium was changed to 1%-2% DMEM.

The PEI-DNA complexes were prepared in the following manner:

a) Preparation of the DNA solution: A DNA solution was prepared with the appropriate quantity of pδF6 (40 μg/plate) and p518 (20 μg/plate) plasmids, and the plasmid that contained the corresponding recombinant AAV-2 genome (20 μg/plate), in saline solution or sterile saline solution, in a final volume of 1 ml per plate. It was mixed and incubated for 5 min at ambient temperature. The plasmids' characteristics are described at the end of the protocol.

b) Preparation of the PEI solution: Simultaneously with the preparation of the DNA solution, the solution of the transfection agent, PEI, was prepared in a final volume of 1 ml of sterile saline solution per plate. In order to calculate the volume of 10 mM PEI that was dissolved in saline solution per plate, we used the equation

$$\mu lPEI = \frac{\mu gDNA \times 3 \times (N/P)}{PEIconcentration},$$

wherein: μg DNA = 80; the N/P quotient = 10; and the PEI concentration = 10mM. The calculated volume of PEI was mixed and incubated for 5 min at amb. T.

c) Once the 5 minutes had elapsed, the PEI solution was added to the DNA and vigorously stirred with a vortex for 15 seconds; subsequently, it was incubated for 30 minutes at ambient temperature in order to allow for the formation and stabilisation of the PEI-DNA complexes.

Subsequently, 2 ml of the PEI-DNA complexes were added per plate, drop by drop, using micropipettes and spreading them throughout the entire plate. The plate was softly stirred in the form of a cross, and incubated at 37°C for 4-6 hours. After this period of time had elapsed, 10 ml of DMEM medium with 5% FBS were added per plate, and it was incubated at 37°C in an atmosphere with 5% $CO_2$, for 48 hours.

At 48 hours post-transfection, the medium was removed from the cells, and they were mechanically removed using a scraper (Costar, Coming). Each plate was washed with 3 ml of clean DMEM medium and collected in a 50-ml Falcon tube. The cells were centrifuged at 1,800 rpm for 5 minutes, and the supernatant was discarded. The total cells were re-suspended in 18.5 ml of clean DMEM medium and frozen at -80 °C for the subsequent purification thereof.

In order to release the virus produced inside the 293T cells, 3 successive freezing and thawing steps at -80°C and 37°C, respectively, were performed. Subsequently, it was centrifuged at 3,000 rpm and 4°C for 5 minutes in order to eliminate the cell residues. The supernatant was incubated with 0.1 mg of DNAse I and RNAse A (Roche) per plate for 30 minutes at 37°C and filtered using filters with a pore size of 0.22 μm (MILLIPORE).

Note: a) the pδF6 plasmid was gently supplied by the AMT (Amsterdam Molecular Therapeutics) company. It contains the necessary adenovirus genes for the viral replication of an AAV, b) the p518 plasmid was gently supplied by the AMT (Amsterdam Molecular Therapeutics) company. It contains the genes that encode the Rep proteins of serotype AAV-2 and the VP proteins of serotype 8.

For the purification of the adeno-associated vectors, the iodixanol gradient ultracentrifugation method was used. In order to prepare the phases, fresh 7.4X PBS-MK buffer was prepared (500 ml of PBS without $Mg^{2+}$ and without $Ca^{2+}$ + 50 ml of 1M $MgCl_2$ + 125 ml of 1M KCl). Table 2 summarises the preparation of the gradient phases used.

Table 2: Composition of the gradient phases used for the purification of the AAVs

| Iodixanol % | Iodixanol | 7.4XPBS-MK | 5M NaCl | Sterile distilled water | Total volume |
|---|---|---|---|---|---|
| 15 | 125 ml | 67.57 ml | 100 ml | 207.43 ml | 500 ml |
| 25 | 125 ml | 40.5 ml | - | 134.5 ml | 300 ml |
| 40 | 200 ml | 40.5 ml | - | 59.5 ml | 300 ml |
| 60 | 60 ml | - | - | - | 60 ml |

The total volumes prepared of each phase depend on the number of purifications to be performed in each case.

All the buffers used in this protocol were sterilised by filtration using filters with a pore size of 0.22 μm (MILLIPORE). For the ultracentrifugation, 25 x 89-mm Quick-Seal-Ultra-Clear tubes (Beckman) were used. The iodixanol gradient was formed using 23-mm glass Pasteur pipettes. A pipette was inserted to the bottom of the tube and preparation of the gradient began by the less dense phase, composed of the cell lysate enriched with the adeno-associated vectors (18.5 ml), followed by 9 ml of the iodixanol solution at 15%, 5 ml of the solution at 25%, 5 ml of the solution at 40% and, finally, 3 ml of the solution at 60%. The tubes were equilibrated and sealed. Subsequently, the ultracentrifugation was performed at 69,000 rpm and 16°C for 1 hour, using the Beckman 70 Ti rotor.

Following the ultracentrifugation, the AAV particles concentrated in the iodixanol 40%-60% interphase were collecting by puncturing the bottom of the tube with a needle and a 5-ml syringe. The 5 ml obtained rich in viral particles (fraction 1), were washed and concentrated in 5% sucrose/PBS, using centricon (Amicon Ultra-15, Centrifugal Filter Devices-MILLIPORE). To this end, the centricons were centrifuged at 5,000 rpm and 4°C for 10 minutes. The number of washings and the centrifugation time vary in each production, and were performed until the absence of viscosity typical of iodixanol was observed. The virus was concentrated in 1 ml of 5% sucrose/PBS, and stored at - 80°C until it was to be used. The percentage of recovery was approximately 94%.

1.2 Measurement of the luciferase activity in a live animal by bioluminescence determination with a CCD camera.

In order to measure the luciferase activity obtained following the administration of the rAAV2/8 virions containing the inducible rAAV-pTet$_{bidi}$-pCMV-luc system, the mice were anaesthesised with ketamine/xylazine and administered 100 $\mu$l of D-luciferin (Xenogen/Alameda, USA) diluted in PBS, at a concentration of 30 mg/ml, by intraperitoneal route. After 5 minutes, the animals were placed in a luminometric camera in the dark (CCD: cooled-charged couple device, IVIS, Xenogen Corp., Alameda, USA) and a luminiscence image was obtained superimposed on a grey-scale photograph. The luminiscence image represents the intensity of light by means of a scale of colours, blue pertaining to the lowest intensity, and red pertaining to the highest intensity. These images were processed using the *LivingImage* computer programme (Xenogen Corp., Alameda, USA), which makes it possible to quantify the signal. The units used to measure the bioluminescence are photons/second. The grounds of the methodology used are described in the following articles: Bronstein I, et al., Chemiluminescent and Bioluminescent Reporter Gene Assays. Anal Biochem, 1994. 219: pp. 196-181; and Contag CH, et al., Advances in in vivo bioluminescence imaging of gene expression. Annu Rev Biomed Eng., 2002. 4: pp. 235-60.

1.3 Basal expression and inducibility of the system in BALB/c mice:

As a first approximation to the characterisation of the rAAV2/8-pTet$_{bidi}$-pCMV-luc system, we injected 3 different doses of virus by intravenous route, in 3 groups of 4 BALB/c females: $1 \times 10^{11}$, $3 \times 10^{10}$, and $1 \times 10^{10}$ vg/mouse. We waited 15 days (since the hepatic transduction by an rAAV8 takes approximately 14 days to achieve its maximum expression (Pañeda, A., et al. Hum. Gene Ther. 2009, 20: 908-917)), and we measured the luciferase activity levels in the absence of the inducer, or basal state expression. At day 15 post-injection, we performed an intraperitoneal (i.p.) injection of 50 mg/kg of dox, and on the following day we maintained the induction by the administration of doxycycline in the drinking water for 6 consecutive days (2 mg/ml of dox, and 5% sucrose). This induction protocol was prepared in our department for another TetON inducible system (Zabala, M., et al., Cancer Res. 2004; 64: 2799-2804), and it was the one wherewith the highest transgene induction levels were obtained, without reaching inducer-mediated toxicity levels.

The luciferase activity measurements are performed by delimiting an area of interest selected by the user. In this case, we selected the upper abdominal, or hepatic, area, represented by a circumference in Figure 3.A, and the animal's total surface area, represented by an oval in Figure 3.B.

The bioluminescence measurements through time are shown in Figure 4.

As may be observed in Figure 4, the total luciferase activity levels, that is, when the entire animal is analysed, are significantly higher than the levels obtained when measuring only the upper abdominal or hepatic area, in both the basal state and the induced state, and during the entire induction period, which indicates that the transgene is expressed to a considerable extent in other organs in addition to the liver, as may also be observed in the bioluminescence images captured by the CCD camera, which are not shown because they have a colour code that cannot be interpreted in a grey scale.

On the other hand, the system's basal or residual activity is dependent on the dose of virus administered. Considering that the background noise of the bioluminescence camera for the selected area is approximately $1 \times 10^5$, we may state that the basal luciferase activity in the hepatic area is relatively low in all three cases, being very close to the background noise with the lowest dose of virus used.

As may be seen in Figure 4, following the administration of doxycycline, an approximately 3- to 4-fold increase in the expression of luciferase is observed in all the groups, in both the hepatic area and the entire animal. On the other hand, it is proven that the expression of the transgene remains stable during the 7 days of induction by the administration of doxycycline in the drinking water.

As regards the system's inducibility kinetics, it is observed that, at 10 hours post-intraperitoneal-administration of doxycycline, the transgene reaches the same level of expression as at 24 hours (without having added the dox to the drinking water yet). Therefore, hereinafter, following the administration of an i.p. injection of doxycycline, the luciferase activity in the induced state will be measured at 24 hours post-administration of the drug.

1.4 <u>Dose-response of the system to doxycycline and toxicity</u>

In view of the fact that the luciferase activity levels obtained in the induced state were very low, the decision was made to increase the dose of inducer. To this end, two consecutive doses of 100 and 200 mg/kg of dox were administered by i.p. route, separated by a 14-day period.

The luciferase activity in the hepatic area was measured at 24 hours post-induction. Upon comparing the luciferase activity levels in the induced state, a linear response with respect to the dose of dox administered is observed (Figure 5), but this linear character is lost when the dose of 200 mg/kg of dox is reached. Upon reaching this point, several mice died and the rest were sacrificed, since they already presented symptoms of dox-mediated toxicity (fever, abdominal adhesions, etc.). These symptoms were also observed in the control mice, those without a vector. The system's maximum rate of induction is reached at a dose of 100 mg/kg of dox, and is approximately 10-fold.

1.5 <u>Biodistribution of rAAV-pTet$_{bidi}$-pCMV-luc</u>

In order to study the biodistribution of the transgene activity obtained following the induction of the rAAV-pTet$_{bidi}$-pCMV-luc system in mice from both strains and sexes, 4-8 mice were induced per group (they had been infected 21 days before with $1 \times 10^{11}$ vg of rAAV2/8/mouse with the corresponding induction system), with 50 mg/kg of i.p. dox. At 24 h, the luciferase activity was determined in *vivo* in the CCD camera and, subsequently, the animals were sacrificed and several of their organs were extracted and immediately frozen in order to later measure the luciferase activity of each and normalise it with the amount of total protein.

In Figure 11, it is clearly observed that, in females from both of the two strains studied (BALB/c and C57BL/6), the rAAV2/8-pTet$_{bidi}$-pCMV-luc system is expressed in the liver, as well as in the heart, the uterus and the ovary, which matches the *in vivo* images collected from the luminometric camera, wherein there is a disperse distribution of bioluminescence throughout the animal that is more intense in the lower abdominal area (data not shown). These data also corroborate the observations from a study with several serotypes of AAV performed in our group (Pañeda, A., et al. Hum. Gene Ther. 2009, 20: 908-917). Moreover, it transduces other organs, such as the pancreas, the skeletal muscle, etc., in agreement with the observations made by several authors in relation to serotype AAV8 with constitutive promoters (Nakai, H., et al. J. Virol, 2005, 79: 214-224; Wang, Z., et al., Nat. Biotechnol. 2005, 23: 321-328).

In the case of males, the biodistribution patterns of luciferase activity in both systems are very similar in both mouse strains (Figure 12), as was observed in the females, and match the bioluminescence images obtained with the CCD camera prior to their being sacrificed (data not shown). We found expression of luciferase primarily in the liver and in the heart, followed by the kidneys, stomach, intestine and muscle.

In conclusion, the rAAV-pTet$_{bidi}$-pCMV-luc virus designed is not suitable to regulate transgene expression in the liver, since its maximum rate of induction is limited (approximately 10), and the biodistribution of the transgene activity is not primarily restricted to the liver, but spreads throughout several animal organs.

**Example 2. Construction and characterisation of the recombinant rAAV-pTet$_{bidi}$-pAlb-luc virus.**

After verifying that the vector with the rAAV-pTet$_{bidi}$-pCMV-luc system exhibited a very poor inducibility and a significant expression outside the liver, we attempted to construct a system that made it possible to achieve, on the one hand, a maximum rate of induction greater than the preceding one and, on the other hand, a localised expression of the transgene in our organ of interest, the liver. To this end, the minimal CMV promoters were replaced with albumin promoters (pAlb). Studies performed by Zabala *et al.* (Zabala, M., et al., Cancer Res. 2004; 64: 2799-2804) had shown that pAlb has a low residual expression (even lower than the minimal pCMV) when it is used in other inducible systems, whereas it exhibits a very high rate of inducibility when it is located next to the TetO7 sites. On the other hand, it was already known that the expression thereof is hepatocyte-specific (Frain, M., et al. Mol Cell Biol, 1990, 10: 991-999; Cereghini, S., et al. Cell, 1987, 50: 627-633). However, the maximum induction levels reached with this promoter preceded by the TetO7 sites were significantly lower than those obtained with the minimal CMV promoter (also preceded by the TetO7 sites), in the context of a tetracycline-inducible system wherein the transactivator was constitutively expressed starting from the CMV promoter (Zabala, M., et al., Cancer Res. 2004; 64: 2799-2804).

This new system is called rAAV2/8-pTet$_{bidi}$-pAlb-luc (Figure 2.B).

2.1 <u>Construction of the vector</u>

The plasmid that contains the recombinant AAV genome with the inducible pTet$_{bidi}$-pAlb-luc system (pAC1M2-pAlb-luc) was generated in the following manner: the fragment containing the 7 tetracycline-responsive operator sites and the albumin promoter (TetO7-pAlb) was amplified from the pTonL2(T)-mIL12 plasmid generated in our department (Zabala, M., et al., Cancer Res. 2004; 64: 2799-2804) with primers C (sense primer) and D (anti-sense primer) (C: AGC GCT TTA CGC GTC GAG TTT ACC ACT (SEQ ID NO: 13); D: GTCGAC TTA GTG GGG TTG ATA GGA AAG (SEQ ID NO: 14)), which contain, at the 5'-ends thereof, the AfeI (sense primer) and SalI (anti-sense primer) sites. This fragment was sub-cloned in the pCDNA3.1/V5-His TOPO TA cloning vector (Invitrogen) and was called: pCDNA3.1-TetO7-pAlb.

On the other hand, the albumin promoter was amplified from the pAlb-luc plasmid (Kramer G. et al. Molecular Therapy 2003, 7: 375-385) with primers E (sense) and F (anti-sense) (E: AGC GCT ACA GCT CCA GAT GGC AAA (SEQ ID NO: 15); F: AGC GCT GAA TTC TTA GTG GGG TTG ATA GGA AAG (SEQ ID NO: 16)), which contain, at the 5'-ends thereof, the AfeI sites (sense primer) and the AfeI sites, and EcoRI (anti-sense primer). This fragment was sub-cloned in the pCDNA3.1/V5-His TOPO TA cloning vector (Invitrogen) and was called: pCDNA3.1-pAlb. The albumin promoter was extracted from this vector by digestion with AfeI, and inserted in the pCDNA3.1-TetO7-pAlb plasmid, which was digested with the same enzyme. Those plasmids the digestion whereof with EcoRI/SalI resulted in a band of approximately 700 pb, corresponding to the pAlb-Tet07-pAlb fragment, were selected. This digestion fragment was inserted in the pAC1M2-pCMV-luc plasmid, digested with the same enzymes, to obtain the pAC1M2-pAlb-luc plasmid.

The production and purification of the rAAV8-pTet$_{bidi}$-pAlb-luc vector was performed in the same manner as that described for the preceding inducible system.

## 2.2 Basal expression and inducibility of the system in BALB/c mice

As a first approximation, 4 female BALB/c mice were injected by intravenous route with a dose of rAAV2/8-pTet$_{bidi}$-pAlb-luc virus of $1 \times 10^{11}$ vg/mouse, and both the basal luciferase activity (at 14 days post-intravenous-injection of the vector) and the induced-state luciferase activity (24 h post-i.p.-administration of 50 mg/kg of dox, and day 22 post-administration of the vector) were measured in the hepatic area and in the total animal. This i.p. dose of dox was used to ensure that no undesireable toxic effects were produced due to an excessive dose of inducer.

Upon comparing the luciferase activity in both the basal state and the induced state, in the upper abdominal area (hepatic) and the total animal, no significant differences were observed between them (Figure 6), indicating that the rAAV2/8-pTet$_{bidi}$pAlb-luc system is specifically expressed in the hepatic area. For this reason, hereinafter, whenever luciferase activity data are shown, they will refer to the measurement in the hepatic area of the mice.

On the other hand, upon comparing the measurement data for the hepatic area and those corresponding to the rAAV2/8-pTet$_{bidi}$-pCMV-luc system (with the same dose of vector, and the same dose of dox administered), it is observed that, in the basal state, the luciferase activity is significantly lower with the rAAV2/8-pTet$_{bidi}$-pAlb-luc system, albeit not negligible (Figure 7).

However, the most outstanding finding is the rate of induction obtained with the new rAAV2/8-pTet$_{bidi}$-pAlb-luc system following the administration of an i.p. dose of 50 mg/kg of dox, which is approximately 250 (Figure 7), almost 85 times higher than the rate of induction of the rAAV2/8-pTet$_{bidi}$-pCMV-luc system, an unexpected finding in light of the results obtained by Zabala *et al.* in a study already cited (Zabala, M., et al., Cancer Res. 2004; 64: 2799-2804). Moreover, the difference between both systems for this dose of dox in the induced state is highly significant.

## 2.3 Long-term re-inducibility of the system

One of the important characteristics that any inducible system must meet is the capacity to be re-induced through time; for this reason, we performed repeated i.p. administrations of dox (50 mg/kg), separated by 15-day intervals (between the 1st and the 2nd, and between the 2nd and the 3rd inductions) and 80 day-intervals (between the 3rd and the 4th). The expression of luciferase was measured at 24 hours post-induction. Figure 8 shows that at doses of both $1 \times 10^{10}$ and $1 \times 10^{11}$ vg/mouse, the system's re-induction capacity is maintained for at least 4 months. However, it may be observed that the maximum rate of induction is dependent on the dose of virus administered, given that the rate obtained with the lowest dose of virus is approximately 10, whereas that obtained with the high dose is 250.

## 2.4 Dose-response to doxycycline in C57B6/L mice, and comparison of the system's inducibility between sexes.

Upon comparing the luciferase levels obtained in the basal state and the induced state with the rAAV2/8-pTet$_{bidi}$-pAlb-luc system in female C57BL/6 mice vs. female BALB/c mice, we observed that both levels are significantly higher in the C57BL/6 females, which suggests that rAAV2/8 transduces the liver of this mouse strain better than the BALB/c strain; however, we did not find significant differences in the rate of induction of the system in this experiment. Hereinafter, the inducible rAAV2/8-pTet$_{bidi}$-pAlb-luc system will continue to be characterised in C57BL/6 mice.

On the one hand, we wished to evaluate the dependency between the transgene activity in the induced state and the dose of inducer administered. The existence of a linear relationship between both parameters allows for a precise control of the transgene expression, which is important when the therapeutic range of the transgene is limited, or when the toxicity thereof requires a strict control of its levels.

On the other hand, given the difference between sexes in the hepatic transduction of rAAV8, it seemed interesting to study its influence on the inducible system. For this reason, the dose response to dox was measured in females and males of the same strain.

To this end, 5 groups of 5 animals each were formed, for each sex, which were injected a dose of rAAV2/8-Tet$_{bidi}$pAlb-luc virus of $1 \times 10^{11}$ vg/mouse. The basal activity was measured at 14 days post-injection of the virus (pre-induction), and, at 21 days post-injection of the vector, an i.p. dose of dox was administered to each group: 2, 10, 25, 50 and 100 mg/kg. The luciferase activity was measured at 24 hours post-induction.

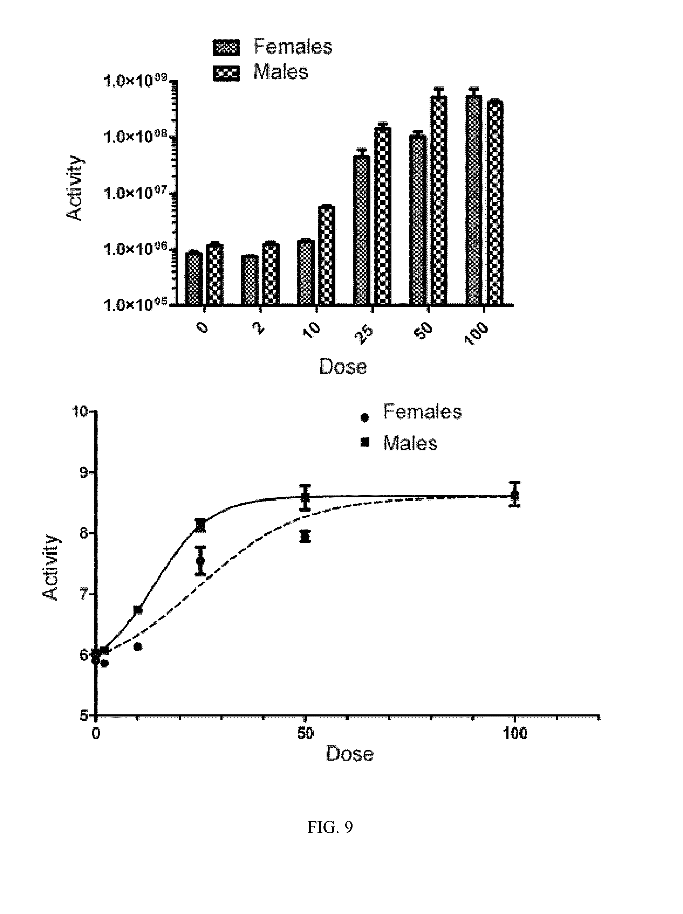

Significant differences are observed in the luciferase levels in the induced state between males and females from doses of 2 mg/kg up to doses of 50 mg/kg of dox, the luciferase activity being greater in the males than in the females (Figure 9A). The maximum rate of induction of the rAAV2/8-pTet$_{bidi}$-pAlb-luc system in this strain is reached at a dose of 100 mg/kg in the case of females (since, at a higher dose, the toxic effects of dox would make it impossible to observe an increase in the luciferase levels), which is 650, whereas in the males it is reached at a dose of 50 mg/kg and is 450.

Following the administration of the dose of 100 mg/kg of dox, it is observed that both males and females reach similar luciferase activity levels. This, jointly with the fact that the males reach the same luciferase activity levels when they are induced with 50 and 100 mg/kg of dox, suggests that the system's saturation state is being reached, wherein all the system operator sites present in the cell are occupied by the transactivator-inducer complex.

Figure 9.B shows the logarithmic values of the luciferase activities observed in Figure 9.A, which fit a sigmoidal curve typical of saturatable systems; this allows us to estimate the luciferase activity at a given dose of inducer within the linear range of each curve (2 to 25 mg/kg of dox for the males, and 10 to 50 mg/kg for the females).

2.5 Evaluation of the oral administration route for the inducer.

Another issue to be borne in mind is the toxicity of the inducer drug as a function of the administration route used. It has been described that the oral administration of dox is much safer than the intraperitoneal administration thereof, in terms of associated toxicity, in animal models. The fact that intraperitoneal administration is used in animal models is due to reproducibility reasons, since the researchers may ensure the correct administration of the calculated dose, whereas oral administration (in the drinking water in the case of small animals) has a higher risk of data dispersion, given the different quantities of water consumed by each animal. In any event, the oral-administration clinical form of this antibiotic also ensures correct dosage of the drug. For all these reasons, we decided to study whether the inducibility of the system was affected by the administration route used for the inducer; to this end, we induced the system by the oral administration of dox in the drinking water (2 mg/ml dox, 5% sucrose). As may be observed in Figure 10, the luciferase activity levels in the induced state obtained by oral administration of the drug are somewhat lower (approximately 50%) than those obtained at 24 hours post-i.p.-induction with 50 mg/kg of dox, which indicates that the oral route may be effectively and safely used to induce this system, although it does not reach the higher levels reached by the highest i.p. doses of dox. Greater concentrations of dox in the drinking water were not assayed; instead, similar protocols to those already described in the literature for tetracycline induction systems were used.

2.6 Biodistribution of rAAV-pTet$_{bidi}$-pAlb-luc.

It had already been observed that the luciferase activity was more restricted to the liver upon comparing the levels measured in the hepatic area versus the total animal (Figure 6) in live animals using a CCD camera.

Now then, in order to perform a more detailed, comprehensive study of the biodistribution of the transgene activity obtained following the induction of the new system in mice from both strains and sexes, we induced 4-8 mice per group (which had been infected 21 days earlier with $1 \times 10^{11}$ vg of rAAV2/8/mouse with the corresponding induction system), with 50 mg/kg of dox i.p. At 24 h, we measured the luciferase activity in *vivo* in the CCD camera and, subsequently, we sacrificed the animals and extracted several of their organs, freezing them immediately thereafter in order to later measure the luciferase activity of each of them and normalise it with the amount of total protein. This protocol is the same that was applied in the case of the preceding inducible system.

In both females and males, luciferase expression is exclusively observed in the liver of animals from both strains with the rAAV-pTet$_{bidi}$-pAlb-luc system (Figures 11 and 12). If each organ is studied, the expression caused by this system is always greater (and, in almost all cases, significantly so) than that caused by the rAAV-pTet$_{bidi}$-pAlb-luc system, with the exception of the liver. In the liver, we observe that the rAAV-pTet$_{bidi}$-pAlb-luc system reaches induction levels of luciferase activity which are greater than the rAAV-pTet$_{bidi}$-pCMV-luc system in both strains, which corroborates the predictions made on the basis of the *in vivo* bioluminescence measurements, performed in the hepatic area with both systems. In this way, the hepato-specificity of the inducible rAAV2/8-pTet$_{bidi}$-pAlb-luc system in female and male mice from both strains is proven.

In conclusion, the rAAV-pTet$_{bidi}$-pAlb-luc system is the first inducible hepato-specific system described for adeno-associated vectors (inducible hepato-specific systems carried by hydrodynamic injection (Zabala, M., et al., Cancer Res. 2004; 64: 2799-2804) and by high-capacity adenoviruses (Wang, L., et al. Gastroenterology, 2004. 126: 278-289), the size whereof is greater than the maximum accepted by rAAVs, have already been described), and represents a very important tool for the long-term treatment of hepatic diseases which require a strict regulation of the expression of the transgene, either because they present a limited therapeutic range (as in the case of IGF-1 in hepatic cirrhosis models) or because they present toxic effects when expressed in an uncontrolled manner (as in the case of several immunoenhancing cytokines, such as IL-12, IFN$\gamma$, IFN$\alpha$, etc.).

**Example 3. Construction and characterisation of the recombinant rAAV-pTet$_{bidi}$-pAlb-IL12 virus.**

Numerous pre-clinical studies showed the anti-tumour efficacy arising from the gene transfer of IL-12. These data drove to perform a phase I clinical trial for the treatment of digestive system tumours using a first-generation adenovirus, carrying IL-12 (Sangro, B., et al. J Clin Oncol. 2004, 22: 1389-1397). The clinical trial showed the need for a more prolonged expression of IL-12 in order to obtain a therapeutic effect. In this regard, our department is working to develop long-term-expression viral vectors that carry inducible IL-12 expression systems (Wang, L., et al. Gastroenterology, 2004. 126: 278-289).

Amongst the viral vectors that we have available, AAV is a very promising candidate, since it is a long-term expression vector and the clinical-grade production thereof at high doses had already been demonstrated (Meghrous, J., et al. Biotechnol Prog. 2005, 21:154-160).

For this reason, we attempted to develop an adeno-associated vector capable of producing IL-12 in an inducible, hepato-specific manner, and to test the anti-tumour activity thereof in animal models.

The implantation of certain colon cancer lines in the liver of syngeneic mice, such as the MC38 line in C57 mice, is a type of intrahepatic cancer model that is widely used in immunotherapy and other pre-clinical therapy approaches (Heijstek, M.W., et al. Dig Surg, 2005, 22: 16-25); therefore, it is the model selected to test the new therapeutic vector.

Single-chain mIL-12 (mIL-12 sc), composed of subunits p40 and p35 fused in a single protein sequence, was used. mIL-12 is much smaller than the construct that is habitually used to express IL-12, which is composed of subunit P35, an IRES (Internal Ribosomal Entry Site) element and subunit P40 (Waehler, R., et al. Hum Gene Ther, 2005, 16: 307-317). This makes it possible to sub-clone it in the viral framework of rAAV and generate the corresponding recombinant virus. Moreover, it has been described that the protein resulting from the expression of this construct is more active than that obtained from the construct wherein both subunits are associated by means of an internal ribosome-binding sequence or IRES.

Therefore, the luciferase gene was replaced with the mIL-12 sc gene, to obtain the construct shown in Figure 2.C. This new construct is called rAAV-pTet$_{bidi}$-pAlb-mIL12. Subsequently, the rAAV2/8-pTet$_{bidi}$-pAlb-mIL12 virus was produced.

3.1 <u>Construction of the vector</u>

In order to generate the pAC1M2-pAlbIL12 plasmid that contains the recombinant AAV genome with the inducible pTet$_{bidi}$-pAlb-mIL12 system, mIL12sc was amplified by PCR from the pCDNA3.1-mIL12sc plasmid (gently supplied by Dr. Crettaz in our department. The construct and the sequence of mIL12sc are detailed in Lieschke, G.J., et al. (Nat. Biotechnol. 1997, 15: 35-40), with primers G (sense) and H (anti-sense) (G: GTC GAC ATG GGT CCT CAG AAG (SEQ ID NO: 17), H: GCG GCC GCT TAG GCG GAG CTC AGA TAG CC (SEQ ID NO: 18)), which contain, at the 5'-ends thereof, the SalI (sense primer) and NotI (anti-sense primer) sites. This fragment was sub-cloned in the pCDNA3.1/V5-His TOPO TA cloning vector (Invitrogen), and was extracted therefrom by digestion with the enzymes mentioned above. The fragment extracted was inserted in the pAC1M2-pAlb-luc vector, previously digested with SalI/ NotI, to obtain the pAC1M2-pAlb-mIL12sc plasmid.

The production and purification of the rAAV2/8-pTet$_{bidi}$-pAlb-mIL12 virus was performed in the same manner as that described for the other vectors already described.

3.2 <u>Anti-tumour protocol.</u>

<u>Description of the tumour model</u>

As mentioned above, we will use the model of hepatic metastasis of colorectal cancer, by implanting MC38 cells in the liver of C57BL/6 syngeneic mice. The method used consists of the hepatic implantation (by laparotomy) of 500,000 MC38 cells in the liver's larger lobe. Seven days after the hepatic implantation of the cells, tumours 4-6 mm in diameter are observed (by laparotomy), which grow without interruption until they cause the death of the mouse about 30-50 days post-implantation.

The following protocol was used: 3 different doses of the rAAV2/8-pTet$_{bidi}$-pAlb-IL12 vector were administered by intravenous route, to 3 groups of female C57BL/6 mouse (N = 5): $3 \times 10^{10}$, $1 \times 10^{10}$ and $3 \times 10^9$ vg/mouse. One month after the injection of the vector, $5 \times 10^5$ MC38 cells were implanted in the liver's larger lobe (5 control mice were added, whereto the vector had not been previously administered), and, 10 days later, an i.p. induction of 50 mg/kg of dox was performed, which was maintained for a week in the drinking water (2 mg/ml of dox) with 5% sucrose. We waited one month before implanting the tumoural cells, in order to prevent a possible immune response against the rAAV8 capsid which might be present shortly following the administration of the vector. Blood was extracted from the mice at several points, and the serum concentration of mIL12sc, mIFNγ and transaminases was measured (Figure 13).

A linear relationship between the levels of mIL-12 sc and mIFNgamma in the induced state and the dose of virus administered was corroborated. The expression of mIL12sc was transitory, and decreased to undetectable levels at day 7 post-induction (inhibition mediated by mIFNgamma, already described by Reboredo, M., et al. (Reboredo, M., et al. Gene Ther. 2008, 15: 277-288)). The levels of mIFNgamma reached an expression peak at day 7 post-induction. The expression of both cytokines was undetectable for the three doses of virus administered in the basal state, prior to the induction, measured by means of ELISA.

### 3.3 Absence of toxicity.

One of the most significant problems upon using IL-12 as an anti-tumoural agent is its high systemic toxicity when the expression is high and not regulated. For this reason, it was decided to analyse the toxic effects caused by the expression of mIL-12 sc by the vector in the liver. To this end, the serum transaminases (AST: aspartate aminotransferase, and ALT: alanine aminotransferase) were measured during and after the induction.

In no case visible toxic effects were observed in the animals, and the serum transaminase measurements indicate the absence of hepatic failure, which is significant at the times selected (Figure 14), although limited, transitory increases in both enzymes are observed, primarily at 24 hours post-i.p.-induction of dox. This indicates that the regulated induction of IL-12 is safe in terms of toxicity.

### 3.4. Profilactic anti-tumoural efficacy: Survival

The first way to measure the anti-tumoural efficacy of the treatment was to determine the percentage of survival of the different groups of mice in time. The experiment was considered to be concluded at day 132 of the protocol, corresponding to day 102 post-implantation of the tumoural cells (Figure 13). The mice which, on this day, were alive did not present intrahepatic tumours; however, all the animals that died during the study presented large-size tumours (approximately 4 cm$^3$). No mortality due to the expression of IL-12 was observed. All the control mice and those injected with the lowest dose of the virus died, although a slight delay in the growth of the tumour was observed in the latter. Figure 15 shows that the minimal therapeutic dose of the rAAV2/8-pTet$_{bidi}$-pAlb-mIL12 virus was $1 \times 10^{10}$ vg/mouse, which led to 100% tumour-free mice. The dose of $3 \times 10^{10}$ vg/mouse produced a percentage of 80% tumour-free mice.

### 3.5 Anti-tumoural efficacy: Evaluation of the memory immune response.

In order to evaluate the efficacy of the memory immune response induced by the administration of the vector, we performed a rechallenge (or second challenge) of MC38 cells ($1 \times 10^6$ cells/mouse) administered subcutaneously to those mice. Figure 16 shows the tumor progression in time for the mice that had been previously treated, as compared to 5 untreated, control mice. Protection is observed in 40% of the previously treated mice, whereas the tumour size at the end of the experiment (day 42 post-rechallenge and day 132 of the protocol) is significantly lower in the groups treated than in the untreated mice. These data indicate the development of an effective effector memory response in the treated mice, as compared to the untreated ones.

On the other hand, the percentage of CD8$^+$-MC38Tet$^+$ PBLs (specific tetramers loaded with immunodominant peptide from the MC38 line) present at day 23 post-rechallenge (day 113 of the protocol) was labelled and analysed in all the groups. It was observed that the group treated, which groups the mice treated with the two highest doses of vector, since there are no significant differences between them, presented significantly higher levels of these lymphocytes than the untreated controls (Figure 17).

Finally, the presence of these effector lymphocytes in the tumour surroundings was measured; to this end, the animals were sacrificed and the CD8$^+$ present in the tumour homogenate was labelled. It was analysed by means of FACS and the FlowJo programme, the percentage of MC38 tetramer-specific CD8+ lymphocytes used above and positive for activation marker CD44. Since no significant differences were observed between the groups treated, the data are shown grouped in Figure 18. Significant differences are observed between the previously treated mice and the controls, which indicates that there are not only effector memory cells in the peripheral blood, but that they are also located in the tumour, where they will perform their cytotoxic function. Approximately 50% of the CD8+ effector lymphocytes are tetramer-specific and are activated in the mice who received the treatment.

In conclusion, it is proven that the inducible rAAV-pTet$_{bidi}$-pAlb system that encodes single-chain mIL12 is a good anti-tumour treatment for hepatic metastasis of colorectal carcinoma, with no toxic effects associated with the expression of IL-12 and the development of an effective cellular response. Moreover, we observe the vector-mediated induction of an efficient memory-type cellular response upon rechallenging the mice treated with the same cells transformed. This could be a possible application for the inducible hepato-specific system presented in this work.

3.6 Therapeutic antitumor efficacy: Survival

After checking that the treatment was able to prevent tumor development, it was checked whether the same treatment was capable of eliminating already implanted tumors. To that end, MC38 tumor cells were first implanted, after seven days a single dose of the therapeutic vector was injected. Seven days after the injection of the vector, the induction of IL-12 expression was started (Figure 19). The experiment was considered ended on day 100 after the implantation of the tumor cells The mice which were alive on that day did not have intrahepatic tumors, however, all the animals which died during the study have tumors of a large size (approximately 4 cm$^3$). No mortality due to IL-12 expression was observed. All the control mice died (Figure 20).

**Claims**

1. A gene construct that allows for the inducible hepato-specific expression of a polynucleotide of interest in response to an inducer agent, which comprises

   (i) an inducible bi-directional operator-promoter that comprises at least one responsive element to said inducer agent flanked by a first hepato-specific promoter sequence and a second hepato-specific promoter sequence, wherein both hepato-specific promoter sequences act in a divergent manner,
   (ii) a first nucleotide sequence which comprises a sequence that encodes a transactivator which may be activated by said inducer agent and a polyadenylation signal located at the 3' position with respect to the region that encodes the transactivator, wherein said sequence that encodes a transactivator is operatively coupled to the first hepato-specific promoter sequence, and
   (iii) a second nucleotide sequence that comprises a polynucleotide that is operatively coupled to the second hepato-specific promoter sequence and a polyadenylation signal located at the 3' position with respect to the polynucleotide of interest,

   wherein the promoter activity of said first and second hepato-specific promoter sequences is induced as a consequence of the binding of the transactivator to the operator region of the operator-promoter in the presence of the inducer agent.

2. A gene construct according to claim 1, wherein the responsive element to the inducer agent comprises at least one tetracycline-responsive element and the transactivator is a reverse tetracycline transactivator.

3. A construct according to claim 2, wherein the tetracycline-responsive element comprises the nucleic acid sequence defined in SEQ ID NO: 1.

4. A gene construct according to any of claims 2 or 3, wherein the reverse tetracycline transactivator is encoded by a polynucleotide that comprises sequence SEQ. ID. NO: 6.

5. A gene construct according to any of claims 1 to 4, wherein the first hepato-specific promoter sequence and the second hepato-specific promoter sequence are identical.

6. A gene construct according to claim 5, wherein the first hepato-specific promoter sequence and the second hepato-specific promoter sequence comprise the albumin gene promoter or a functionally equivalent variant thereof.

7. A gene construct according to claim 6, wherein the albumin gene promoter comprises a sequence selected from the group of SEQ ID NO: 2 and SEQ ID NO: 3.

8. A gene construct according to claim 7, wherein the inducible bi-directional operator-promoter comprises SEQ ID NO: 4.

9. A gene construct according to any of claims 1 to 8, wherein at least one of the polyadenylation signals is a bi-directional polyadenylation signal.

10. A gene construct according to claim 9, wherein the polyadenylation signal is a bi-directional polyadenylation signal from the SV40 virus.

11. A gene construct according to any of claims 1 to 10, wherein the polynucleotide of interest encodes the heavy chain and/or the light chain of IL-12 or a functionally equivalent variant thereof.

12. A gene construct according to claim 11, wherein the polynucleotide of interest encodes a single-chain IL-12.

13. A vector that comprises a gene construct according to any of claims 1 to 12.

14. A recombinant viral genome that comprises a gene construct according to any of claims 1 to 12.

15. A viral genome according to claim 14, wherein said genome is from a recombinant adeno-associated virus.

16. A virion obtainable by expressing a viral genome according to claims 14 or 15, in an adequate packaging cell.

17. An *in vitro* method for the expression of a polynucleotide of interest in a cell of hepatic origin, which comprises the following steps:

(i) placing said cell in contact with a gene construct according to any of claims 1 to 12, with a vector according to claim 13, with a viral genome according to any of claims 14 or 15, or with a virion according to claim 15, under adequate conditions for the entry of said construct, said vector or said virion into the cell, and
(ii) putting the cell in contact with the inducer agent for the necessary time for the expression of the polynucleotide of interest to take place.

18. A pharmaceutical composition that comprises a gene construct according to any of claims 1 to 12, a vector according to claim 13, a viral genome according to claims 14 or 15, or a virion according to claim 16, and a pharmaceutically acceptable carrier.

19. A gene construct according to any of claims 1 to 12, a vector according to claim 13, a viral genome according to claims 14 or 15, a virion according to claim 16, or a pharmaceutical composition according to claim 18, to be used as a medicament.

20. A gene construct according to any of claims 1 to 12, a vector according to claim 13, a viral genome according to claims 14 or 15, a virion according to claim 16, or a pharmaceutical composition according to claim 18, to be used in the treatment of a hepatic disease.

21. A gene construct according to any of claims 1 to 12, a vector according to claim 13, a viral genome according to claims 14 or 15, a virion according to claim 16, or a pharmaceutical composition according to claim 18, wherein the polynucleotide of interest encodes IL-12 or a functionally equivalent variant thereof, to be used in the treatment of hepatic cancer.

22. An inducible bi-directional operator-promoter suitable for the inducible hepato-specific expression of two polynucleotides of interest by an inducer agent, which comprises

(i) at least one responsive element to said inducer agent,
(ii) a first hepato-specific promoter sequence and
(iii) a second hepato-specific promoter sequence,

wherein the first and the second hepato-specific promoter sequences act in a divergent manner with respect to the responsive element to the inducer agent, and wherein the promoter activity of the first and the second hepato-specific promoter sequences increases in the presence of said inducer agent and in the presence of a transactivator that binds to the responsive element.

23. An inducible bi-directional operator according to claim 22, wherein the responsive element to the inducer agent in the regulable bi-directional operator-promoter comprises at least one tetracycline-responsive element.

24. An inducible bi-directional operator according to claim 23, wherein the tetracycline-responsive element comprises a nucleic acid sequence defined in SEQ ID NO: 1.

25. An inducible bi-directional operator according to any of claims 22 to 24, wherein the first hepato-specific promoter sequence and the second hepato-specific promoter sequence are identical.

26. An inducible bi-directional operator according to claim 25, wherein the first hepato-specific promoter sequence and the second hepato-specific promoter sequence comprise the albumin gene promoter or a functionally equivalent variant thereof.

27. An inducible bi-directional operator according to claim 26, wherein the albumin gene promoter comprises a sequence selected from the group of SEQ ID NO: 2 and SEQ ID NO: 3.

28. An inducible bi-directional operator according to claim 27, wherein the nucleotide sequence of the inducible bi-directional operator-promoter comprises SEQ ID NO: 4.

29. A gene construct suitable for the inducible and hepato-specific expression of a polynucleotide of interest by an inducer agent, which comprises

   (a) An inducible bi-directional operator-promoter that comprises

      (i) at least one responsive element to said inducer agent,
      (ii) a first hepato-specific promoter sequence and
      (iii) a second hepato-specific promoter sequence,

   (b) a nucleotide sequence that encodes a transactivator which may be activated by said inducer agent that is operatively coupled to the first hepato-specific promoter sequence and a polyadenylation signal located at the 3' position with respect to the region that encodes the transactivator,

   wherein the first and the second hepato-specific promoter sequences act in a divergent manner with respect to the responsive element to the inducer agent, and wherein the promoter activity of the first and the second hepato-specific promoter sequences increases in the presence of said inducer agent and in the presence of a transactivator that binds to the responsive element in the inducible bi-directional operator-promoter.

30. A gene construct according to claim 29, wherein the responsive element to the inducer agent in the regulable bi-directional operator-promoter comprises at least one tetracycline-responsive element and the transactivator may be activated by said tetracycline.

31. A construct according to claim 30, wherein the tetracycline-responsive element comprises a nucleic acid sequence defined in SEQ ID NO: 1.

32. A gene construct according to any of claims 30 or 31, wherein the reverse tetracycline-inducible transactivator is encoded by a polynucleotide that comprises sequence SEQ ID NO: 2.

33. A gene construct according to any of claims 29 to 32, wherein the first hepato-specific promoter sequence and the second hepato-specific promoter sequence are identical.

34. A gene construct according to claim 33, wherein the first hepato-specific promoter sequence and the second hepato-specific promoter sequence comprise the albumin gene promoter or a functionally equivalent variant thereof.

35. A gene construct according to claim 34, wherein the albumin gene promoter comprises a sequence selected from the group of SEQ ID NO: 2 and SEQ ID NO: 3.

36. A gene construct according to claim 35, wherein the nucleotide sequence of the inducible bi-directional operator-promoter comprises SEQ ID NO: 4.

**37.** A gene construct according to any of claims 29 to 36, wherein the polyadenylation signal is a bi-directional polyadenylation signal.

**38.** A gene construct according to claim 37, wherein the polyadenylation signal is a bi-directional polyadenylation signal from the SV40 virus.

FIG. 1

A

B

FIG. 2

FIG. 3

FIG. 4

FIG. 5

FIG. 6

FIG. 7

FIG. 8

FIG. 9

FIG. 10

FIG. 11

FIG. 12

| Injection of<br>$3 \times 10^{10}$ gv/mouse<br>$1 \times 10^{10}$ gv/mouse<br>$3 \times 10^9$ gv/mouse<br>Virus-free control<br>(N = 5) | Intrahepatic<br>implantation<br>$5 \times 10^5$ MC38<br>cells | Induction<br>50 mg/kg<br>dox i.p. | Dox in drinking<br>water<br>(2 mg/ml) |

0    14    30    40 41    44    47

| Rechallenge<br>$1 \times 10^6$ cells/mouse | Extraction<br>PBLs | sacrifice<br>† |

90    103    113    132

FIG. 13

45

FIG. 14

FIG.15

FIG. 16

FIG. 17

A)

B)

C)

FIG. 18

FIG. 19

FIG. 20

## INTERNATIONAL SEARCH REPORT

International application No

PCT/ES2010/070715

**A. CLASSIFICATION OF SUBJECT MATTER**

INV. C12N15/63    C12N15/86
ADD.

According to International Patent Classification (IPC) or to both national classification and IPC

**B. FIELDS SEARCHED**

Minimum documentation searched (classification system followed by classification symbols)

C12N

Documentation searched other than minimum documentation to the extent that such documents are included in the fields searched

Electronic data base consulted during the international search (name of data base and, where practical, search terms used)

EPO-Internal, BIOSIS, COMPENDEX, Sequence Search, EMBASE, FSTA, WPI Data

**C. DOCUMENTS CONSIDERED TO BE RELEVANT**

| Category* | Citation of document, with indication, where appropriate, of the relevant passages | Relevant to claim No. |
|---|---|---|
| Y | CHTARTO A ET AL: "Tetracycline-inducible transgene expression mediated by a single AAV vector", GENE THERAPY, MACMILLAN PRESS LTD., BASINGSTOKE, GB, vol. 10, no. 1, 1 January 2003 (2003-01-01), pages 84-94, XP002540413, ISSN: 0969-7128, DOI: DOI:10.1038/SJ.GT.3301838 cited in the application the whole document, in particular Fig. 1d ----- -/-- | 1-38 |

[X] Further documents are listed in the continuation of Box C.     [ ] See patent family annex.

* Special categories of cited documents :

"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art.
"&" document member of the same patent family

| Date of the actual completion of the international search | Date of mailing of the international search report |
|---|---|
| 21 February 2011 | 10/03/2011 |

| Name and mailing address of the ISA/ | Authorized officer |
|---|---|
| European Patent Office, P.B. 5818 Patentlaan 2 NL - 2280 HV Rijswijk Tel. (+31-70) 340-2040, Fax: (+31-70) 340-3016 | Bassias, Ioannis |

Form PCT/ISA/210 (second sheet) (April 2005)

## INTERNATIONAL SEARCH REPORT

| | International application No |
|---|---|
| | PCT/ES2010/070715 |

C(Continuation).   DOCUMENTS CONSIDERED TO BE RELEVANT

| Category* | Citation of document, with indication, where appropriate, of the relevant passages | Relevant to claim No. |
|---|---|---|
| Y | CHTARTO ET AL:  "Controlled delivery of glial cell line-derived neurotrophic factor by a single tetracycline-inducible AAV vector", EXPERIMENTAL NEUROLOGY, ACADEMIC PRESS, NEW YORK, NY, US, vol. 204, no. 1, 3 March 2007 (2007-03-03) , pages 387-399, XP005927105, ISSN: 0014-4886, DOI: DOI:10.1016/J.EXPNEUROL.2006.11.014 cited in the application the whole document, in particular Fig. 1C ----- | 1-38 |
| Y | FECHNER ET AL:  "A bidirectional Tet-dependent promotor construct regulating the expression of E1A for tight control of oncolytic adenovirus replication", JOURNAL OF BIOTECHNOLOGY, ELSEVIER SCIENCE PUBLISHERS, AMSTERDAM, NL, vol. 127, no. 4, 22 December 2006 (2006-12-22), pages 560-574, XP005810970, ISSN: 0168-1656, DOI: DOI:10.1016/J.JBIOTEC.2006.09.011 the whole document, in particular Fig. 1 ----- | 1-38 |
| Y | BARON U ET AL:  "CO-REGULATION OF TWO GENE ACTIVITIES BY TETRACYCLINE VIA A BIDIRECTIONAL PROMOTER", NUCLEIC ACIDS RESEARCH, OXFORD UNIVERSITY PRESS, SURREY, GB, vol. 23, no. 17, 11 September 1995 (1995-09-11), XP000775822, ISSN: 0305-1048 the whole document, in particular Fig. 1 ----- | 1-38 |
| Y | ZABALA MAIDER ET AL:  "Optimization of the Tet-on system to regulate interleukin 12 expression in the liver for the treatment of hepatic tumors", CANCER RESEARCH, vol. 64, no. 8, 15 April 2004 (2004-04-15) , pages 2799-2804, XP002624074, ISSN: 0008-5472 cited in the application the whole document ----- -/-- | 1-38 |

## INTERNATIONAL SEARCH REPORT

| International application No |
| --- |
| PCT/ES2010/070715 |

| C(Continuation). DOCUMENTS CONSIDERED TO BE RELEVANT | | |
| --- | --- | --- |
| Category* | Citation of document, with indication, where appropriate, of the relevant passages | Relevant to claim No. |
| Y | WANG ET AL: "Prolonged and inducible transgene expression in the liver using gutless adenovirus: A potential therapy for liver cancer", GASTROENTEROLOGY, ELSEVIER, PHILADELPHIA, PA, vol. 126, no. 1, 1 January 2004 (2004-01-01), pages 278-289, XP005313326, ISSN: 0016-5085, DOI: DOI:10.1053/J.GASTRO.2003.10.075 cited in the application the whole document ----- | 1-38 |
| Y | KRAMER M GABRIELA ET AL: "In vitro and in vivo comparative study of chimeric liver-specific promoters.", MOLECULAR THERAPY : THE JOURNAL OF THE AMERICAN SOCIETY OF GENE THERAPY MAR 2003 LNKD- PUBMED:12668133, vol. 7, no. 3, March 2003 (2003-03), pages 375-385, XP002624075, ISSN: 1525-0016 the whole document ----- | 1-38 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0031135 A
- WO 200519244 A
- WO 0393293 A
- WO 9624676 A
- WO 0028061 A
- WO 9961601 A
- WO 9811244 A
- US 6156303 A
- US 5478745 A, Salmulski
- WO 0028004 A
- US 2003148506 A
- US 20080008690 A
- US 5837484 A
- WO 9827207 A
- US 5658785 A
- WO 9617947 A

- US 6001650 A
- US 5589377 A
- US 5871982 A
- US 6251677 B
- US 6387368 B
- DE 19644500041
- US 6723551 B
- US 20040197895 A
- US 6103526 A
- US 6146874 A
- US 4970154 A
- WO 9640958 A
- US 5679559 A
- US 5676954 A
- US 5593875 A
- WO 07069090 A

**Non-patent literature cited in the description**

- **Yang, Y.W., J. et al.** *Gene Med.,* 2003, vol. 5, 417-424
- **Kramer, M. G. et al.** *Mol. Ther.,* 2003, vol. 7, 375-385
- **Wang, L. et al.** *Proc. Natl. Acad. Sci,* 1999, vol. 96, 3906-3910
- **Wang et al.** *Nat. Biotechnol.,* 1997, vol. 15, 239-43
- **Zabala et al.** *Cancer Research,* 2004, vol. 64, 2799-2804
- **Kramer et al.** *Molecular Therapy,* 2003, vol. 7, 375-385
- **Chtarto et al.** *Gene Therapy,* 2003, vol. 10, 84-94
- **Chtarto et al.** *Experimental Neurology,* 2007, vol. 204, 387-399
- **Lieschke, G.J. et al.** *m Nat Biotechnol,* 1997, vol. 15, 35-40
- **Zabala, M. et al.** *Cancer Res.,* 2004, vol. 64, 2799-2804
- **Baron et al.** *Nucleic Acids Res.,* 1995, vol. 17, 3605-3606
- **Gossen et al.** *Science,* 1995, vol. 278, 1766-1769
- **TiProD.** *Nucleic Acids Research,* 2006, vol. J4, D104-D107
- **Scott et al.** *Biochim. Biophys. Acta,* 1989, vol. 989, 25-48

- **Rosenfeld et al.** *Genes Dev.,* 1991, vol. 5, 897-907
- **Gossen ; Bujard.** *Proc. Natl. Acad. Sci. USA,* 1992, vol. 89, 5547-5551
- **Gossen et al.** *Science,* 1995, vol. 268, 1766-1769
- **No et al.** *Proc. Natl. Acad. Sci. USA,* 1996, vol. 93, 3346-3351
- **Palli et al.** *Eur. J. Biochem.,* 2003, vol. 270, 1308-1315
- **Ho et al.** *Nature,* 1996, vol. 382, 822-826
- **Amara et al.** *Proc. Natl. Acad. Sci. USA,* 1997, vol. 94, 10618-10623
- **Zhao et al.** *Hum. Gene Ther.,* 2003, vol. 14, 1619-1629
- **Seipel, K. et al.** *EMBO J.,* 1992, vol. 13, 4961-4968
- **Triezenberg, S. J. et al.** *Genes Dev.,* 1988, vol. 2, 718-729
- **Baron et al.** *Nucleics Acids. Res.,* 1997, vol. 25, 2723-2729
- **Hlavka ; Boothe et al.** The Tetracyclines'', in ''Handbook of Experimental Pharmacology. Springer-Verlag, 1985, vol. 78
- The Chemistry of the Tetracycline Antibiotics. **L. A. Mitscher.** Medicinal Research. Dekker, N.Y, 1978, vol. 9

- Tetracycline Manufacturing Processes. Chemical Process Reviews. Noyee Development Corporation, 1969, vol. 2
- The Technology of the Tetracyclines. **R. C. Evans.** Biochemical Reference Series 1. Quadrangle Press, 1968
- Tetracycline. **H. F. Dowling.** Antibiotic Monographs, no. 3, Medical Encyclopedia, New York. 1955
- **Urlinger, S. et al.** *Proc. Natl. Acad. Sci USA,* 2000, vol. 97, 7963-7968
- **Gubler et al.** *Proceedings of the National Academy of Sciences, USA,* 1991, vol. 88, 4143
- **Lieschke G.J. et al.** *Nat Biotechnol.,* 1997, vol. 15, 35-40
- NCBI NLM NIH Bethesda. **Altschul, S. et al.** BLAST-Manual
- **Altschul, S. et al.** *J Mol Biol,* 1990, vol. 215, 403-410
- **Zabala, M. et al.** *J Hepatology,* 2007, vol. 47 (6), 807-815
- **Fields et al.** Virology. Lippincott-Raven Publishers, vol. 2
- **Gao et al.** *J. Virology,* 2004, vol. 78, 6381-6388
- **Moris et al.** *Virology,* 2004, vol. 33, 375-383
- **Srivistava et al.** *J. Virology,* 1983, vol. 45, 555
- **Chiorini et al.** *J. Virology,* 1998, vol. 71, 6823
- **Chiorini et al.** *J. Virology,* 1999, vol. 73, 1309
- **Bantel-Schaal et al.** *J. Virology,* 1999, vol. 73, 939
- **Xiao et al.** *J. Virology,* 1999, vol. 73, 3994
- **Muramatsu et al.** *Virology,* 1996, vol. 221, 208
- **Shade et al.** *J. Virol.,* 1986, vol. 58, 921
- **Gao et al.** *Proc. Nat. Acad. Sci. USA,* 2002, vol. 99, 11854
- **Chao et al.** *Molecular Therapy,* 2000, vol. 2, 619
- **Bantel-Schaal et al.** *J. Virol.,* 1999, vol. 73, 939-947
- **Nathwani et al.** *Blood,* 2007, vol. 109, 1414-1421
- **Kitajima et al.** *Atherosclerosis,* 2006, vol. 186, 65-73
- **Russell.** *J. Gen. Virol.,* 2000, vol. 81, 2573-2604
- **Zaldumbide ; Hoeben.** *Gene Therapy,* 2008, 239-246
- **Gao et al.** *Human Gene Therapy,* 1998, vol. 9, 2353
- **Inoue et al.** *J. Virol.,* 1998, vol. 72, 7024
- **Urabe et al.** *Hum. Gene Ther.,* 2002, vol. 13, 1935-1943
- **Potter et al.** *Methods Enzymol.,* 2002, vol. 346, 413-430
- **Muyldermans.** *Biotechnol.,* 2001, vol. 74, 277-302
- Tratado de Farmacia Galénica. 1993
- Remington's Pharmaceutical Sciences. Williams & Wilkins PA, 2000
- **Templeton.** *DNA Cell Biol.,* 2002, vol. 21, 857-867
- **Lindgren, A. et al.** *Trends Pharmacol. Sci,* 2000, vol. 21, 99-103
- **Schwarze, S.R. et al.** *Trends Pharmacol. Sci.,* vol. 21, 45-48
- **Lundberg, M. et al.** *Mol. Therapy,* 2003, vol. 8, 143-150
- **Snyder, E.L. ; Dowdy, S.F.** *Pharm. Res.,* 2004, vol. 21, 389-393
- **Liu, F. et al.** *Gene Ther,* 1999, vol. 6, 1258-66
- **Liu et al.** *Science,* 1999, vol. 305, 1437-1441
- **Hodges et al.** *Exp. Opin. Biol. Ther,* 2003, vol. 3, 91-918
- **Wen et al.** *World J. Gastroenterol,* 2004, vol. 10, 244-9
- **Murao et al.** *Pharm. Res.,* 2002, vol. 19, 1808-14
- **Lin et al.** *Gene Ther.,* 2003, vol. 10, 180-7
- **Hong et al.** *J. Pharm. Pharmacol.,* 2003, vol. 54, 51-8
- **Herrmann et al.** *Arch Virol,* 2004, vol. 149, 1611-7
- **Matsuno et al.** *Gene Ther,* 2003, vol. 10, 1559-66
- **Chtarto, A. et al.** *Gene Ther,* 2003, vol. 10, 84-94
- **Kramer G. et al.** *Molecular Therapy,* 2007, vol. 7, 375-385
- **Chtarto, A. et al.** *Exp Neurol,* 2007, vol. 204, 387-399
- **Bronstein I et al.** Chemiluminescent and Bioluminescent Reporter Gene Assays. *Anal Biochem,* 1994, vol. 219, 196-181
- **Contag CH et al.** Advances in in vivo bioluminescence imaging of gene expression. *Annu Rev Biomed Eng.,* 2002, vol. 4, 235-60
- **Pañeda, A. et al.** *Hum. Gene Ther.,* 2009, vol. 20, 908-917
- **Nakai, H. et al.** *J. Virol,* 2005, vol. 79, 214-224
- **Wang, Z. et al.** *Nat. Biotechnol.,* 2005, vol. 23, 321-328
- **Frain, M. et al.** *Mol Cell Biol,* 1990, vol. 10, 991-999
- **Cereghini, S. et al.** *Cell,* 1987, vol. 50, 627-633
- **Kramer G. et al.** *Molecular Therapy,* 2003, vol. 7, 375-385
- **Wang, L. et al.** *Gastroenterology,* 2004, vol. 126, 278-289
- **Sangro, B. et al.** *J Clin Oncol.,* 2004, vol. 22, 1389-1397
- **Meghrous, J. et al.** *Biotechnol Prog.,* 2005, vol. 21, 154-160
- **Heijstek, M.W. et al.** *Dig Surg,* 2005, vol. 22, 16-25

- **Waehler, R. et al.** *Hum Gene Ther,* 2005, vol. 16, 307-317
- **Lieschke, G.J. et al.** *Nat. Biotechnol.,* 1997, vol. 15, 35-40
- **Reboredo, M. et al.** *Gene Ther.,* 2008, vol. 15, 277-288